# EUROPEAN PATENT SPECIFICATION

(11) **EP 3 481 925 B1**
(45) Date of publication and mention of the grant of the patent: **01.01.2025**
(21) Application number: 17740254.2
(22) Date of filing: 07.07.2017
(51) Int. Cl.: C10M 107/24, A61Q 9/02, C10M 111/04

(54) **LUBRICATING MEMBERS FOR RAZOR CARTRIDGES COMPRISING A METATHESIZED UNSATURATED POLYOL ESTER**
SCHMIERELEMENTE FÜR RASIERKÖPFE MIT EINEM METATHESIERTEN UNGESÄTTIGTEN POLYOLESTER
ÉLÉMENTS LUBRIFIANTS POUR CARTOUCHES DE RASOIR COMPRENANT UN ESTER DE POLYOL INSATURÉ AYANT SUBI UNE MÉTATHÈSE

(30) Priority: 08.07.2016 US 201662359785 P
(43) Date of publication of application: 15.05.2019
(73) Proprietor: The Gillette Company LLC, Boston, MA 02127 (US)
(72) Inventor: ZANNONI, Luke Andrew, Cincinnati, OH 45202 (US); SCHUBERT, Beth Ann, Cincinnati, OH 45202 (US); PANANDIKER, Rajan Keshav, Cincinnati, OH 45202 (US); KEMPER, Joseph Jay, Cincinnati, OH 45202 (US); STRIFE, Robert John, Cincinnati, OH 45202 (US); MOTLAGH, Safa, Cincinnati, OH 45202 (US); SCHEIBEL, Jeffrey John, Cincinnati, OH 45202 (US); STEPHENS, Alison Fiona, Egham Surrey TW20 9NW (GB); SAWIN, Philip Andrew, Cincinnati, OH 45202 (US)
(74) Representative: P&G Patent Germany
(86) International application number: PCT/US2017/041018
(87) International publication number: WO 2018/009743

(56) References cited:
- WO-A1-2017/011253
- US-A1- 2008 060 201
- US-A1- 2013 280 193
- US-A1- 2014 302 103

## Description

### FIELD OF THE INVENTION

The invention relates to for razor cartridges comprising a metathesized unsaturated polyol ester, a lipophilic structurant and optionally a water soluble polymer.

### BACKGROUND OF THE INVENTION

The use of shaving aids in combination with razor blades to provide lubrication benefits during the shave is known. *See e.g.,* U.S. 7,121,754; U.S. 6,298,558; U.S. 5,711,076; U.S.5,134,775; U.S.6,301,785, U.S. 2009/0223057, U.S. 2006/0225285, WO2007/031793 and U.S.5,431,906. Such shaving aids typically comprise a water-insoluble matrix material to provide structural integrity and a water-soluble polymer, such as polyethylene oxide (polyox), in order to provide lubrication during the shave once the water-soluble polymer forms a solution with the water present during shaving. Since the introduction of polyox as a shaving lubricant, however little development has been made in the field, even though polyethylene oxide polymers are not without limitations. There is a desire to extend the benefits provided by shaving aids beyond lubrication to also provide skin benefits during and after the shaving process. Typically this would be achieved through the addition of skin care oils and enabling deposition of them during the shaving process. For example U.S. 6442839 and U.S. 2007/0110703 describe the use of low levels of mineral and essential oils, butters, waxes and silicones. The use of mineral oil to enhance the glide performance is described in U.S. 2008/0060201. However the art also discloses that the presence of oils results in a reduction of the swelling and solubility of the shaving aid contained in water insoluble polymer matrix. The ability of the shaving aid to swell in contact with water is however believed to be the key mechanism by which the lubrication benefit is delivered to the skin. Hence this is not desirable, as it will negatively impact the overall performance. Thus oils are typically avoided in the matrix.

Another limitation of such shaving aids is related to the manufacturing process which typically involves an injection molding or extrusion process step. These processes require the materials to be conveyed through the process at elevated temperatures and shear. The addition of oils in particular causes barrel slip and conveying inconsistencies which is undesirable. These process temperatures can also result in undesirable degradation of the oils.

Consequently there is still a need to provide a solid shaving aid for razor cartridges comprising a liquid phase contained therein exhibiting lubricating and skin care properties over a sustained period which can be readily manufactured without impacting performance due to thermal degradation of the ingredients and which can accommodate additional additives to provide desirable skin care benefits, especially in the liquid form such as oils, and optionally a water soluble polymer.

A wide variety of skin care oils are available commercially. One such desirable oil is metathesised unsaturated polyol esters. The Applicants recognized that the problems with commercially available metathesized unsaturated polyol esters lay in the rheology of such materials as such rheology resulted in a range of spreading that was insufficient with a first class of materials and excessive spreading with a second class of materials. Thus, both classes of commercially available materials exhibited insufficient spreading leading to poor lubrication and feel during the shaving process. Versions of metathesized unsaturated polyol esters are disclosed that have the correct rheology. Such species of metathesized unsaturated polyol esters provide unexpectedly improved skin conditioning benefits after the shave and lubrication during the shave. Additionally these materials are tolerant to the wide range of conditions experienced during the shave such as the high pH from shaving gels and foams and provide synergistic benefits in the presence of cationic polymeric materials.

### SUMMARY OF THE INVENTION

The invention provides a lubricating member in accordance with the claims. Unless stated otherwise all % are given as weight % of the lubricating member.

### DETAILED DESCRIPTION OF THE INVENTION

### Definitions

The terms "natural oils," "natural feedstocks," or "natural oil feedstocks" may refer to oils derived from plants or animal sources. The term "natural oil" includes natural oil derivatives, unless otherwise indicated. The terms also include modified plant or animal sources (e.g., genetically modified plant or animal sources), unless indicated otherwise. Examples of natural oils include, but are not limited to, vegetable oils, algae oils, fish oils, animal fats, tall oils, derivatives of these oils, combinations of any of these oils, and the like. Representative non-limiting examples of vegetable oils include canola oil, rapeseed oil, coconut oil, corn oil, cottonseed oil, olive oil, palm oil, peanut oil, safflower oil, sesame oil, soybean oil, sunflower oil, linseed oil, palm kernel oil, tung oil, jatropha oil, mustard oil, pennycress oil, camelina oil, and castor oil. Representative non-limiting examples of animal fats include lard, tallow, poultry fat, yellow grease, and fish oil. Tall oils are by-products of wood pulp manufacture.

The term "natural oil derivatives" refers to derivatives thereof derived from natural oil. The methods used to form these natural oil derivatives may include one or more of addition, neutralization, overbasing, saponification, transesterification, esterification, amidification, hydrogenation, isomerization, oxidation, alkylation, acylation, sulfurization, sulfonation, rearrangement, reduction, fermentation, pyrolysis, hydrolysis, liquefaction, anaerobic digestion, hydrothermal processing, gasification or a combination of two or more thereof. Examples of natural derivatives thereof may include carboxylic acids, gums, phospholipids, soapstock, acidulated soapstock, distillate or distillate sludge, fatty acids, fatty acid esters, as well as hydroxy substituted variations thereof, including unsaturated polyol esters. In some embodiments, the natural oil derivative may comprise an unsaturated carboxylic acid having from about 5 to about 30 carbon atoms, having one or more carbon-carbon double bonds in the hydrocarbon (alkene) chain. The natural oil derivative may also comprise an unsaturated fatty acid alkyl (e.g., methyl) ester derived from a glyceride of natural oil. For example, the natural oil derivative may be a fatty acid methyl ester ("FAME") derived from the glyceride of the natural oil. In some embodiments, a feedstock includes canola or soybean oil, as a non-limiting example, refined, bleached, and deodorized soybean oil (i.e., RBD soybean oil).

The term "free hydrocarbon" refers to any one or combination of unsaturated or saturated straight, branched, or cyclic hydrocarbons in the C₂ to C₂₂ range.

The term "metathesis monomer" refers to a single entity that is the product of a metathesis reaction which comprises a molecule of a compound with one or more carbon-carbon double bonds which has undergone an alkylidene unit interchange via one or more of the carbon-carbon double bonds either within the same molecule (intramolecular metathesis) and/or with a molecule of another compound containing one or more carbon-carbon double bonds such as an olefin (intermolecular metathesis).

The term "metathesis dimer" refers to the product of a metathesis reaction wherein two reactant compounds, which can be the same or different and each with one or more carbon-carbon double bonds, are bonded together via one or more of the carbon-carbon double bonds in each of the reactant compounds as a result of the metathesis reaction.

The term "metathesis trimer" refers to the product of one or more metathesis reactions wherein three molecules of two or more reactant compounds, which can be the same or different and each with one or more carbon-carbon double bonds, are bonded together via one or more of the carbon-carbon double bonds in each of the reactant compounds as a result of the one or more metathesis reactions, the trimer containing three bonded groups derived from the reactant compounds.

The term "metathesis tetramer" refers to the product of one or more metathesis reactions wherein four molecules of two or more reactant compounds, which can be the same or different and each with one or more carbon-carbon double bonds, are bonded together via one or more of the carbon-carbon double bonds in each of the reactant compounds as a result of the one or more metathesis reactions, the tetramer containing four bonded groups derived from the reactant compounds.

The term "metathesis pentamer" refers to the product of one or more metathesis reactions wherein five molecules of two or more reactant compounds, which can be the same or different and each with one or more carbon-carbon double bonds, are bonded together via one or more of the carbon-carbon double bonds in each of the reactant compounds as a result of the one or more metathesis reactions, the pentamer containing five bonded groups derived from the reactant compounds.

The term "metathesis hexamer" refers to the product of one or more metathesis reactions wherein six molecules of two or more reactant compounds, which can be the same or different and each with one or more carbon-carbon double bonds, are bonded together via one or more of the carbon-carbon double bonds in each of the reactant compounds as a result of the one or more metathesis reactions, the hexamer containing six bonded groups derived from the reactant compounds.

The term "metathesis heptamer" refers to the product of one or more metathesis reactions wherein seven molecules of two or more reactant compounds, which can be the same or different and each with one or more carbon-carbon double bonds, are bonded together via one or more of the carbon-carbon double bonds in each of the reactant compounds as a result of the one or more metathesis reactions, the heptamer containing seven bonded groups derived from the reactant compounds.

The term "metathesis octamer" refers to the product of one or more metathesis reactions wherein eight molecules of two or more reactant compounds, which can be the same or different and each with one or more carbon-carbon double bonds, are bonded together via one or more of the carbon-carbon double bonds in each of the reactant compounds as a result of the one or more metathesis reactions, the octamer containing eight bonded groups derived from the reactant compounds.

The term "metathesis nonamer" refers to the product of one or more metathesis reactions wherein nine molecules of two or more reactant compounds, which can be the same or different and each with one or more carbon-carbon double bonds, are bonded together via one or more of the carbon-carbon double bonds in each of the reactant compounds as a result of the one or more metathesis reactions, the nonamer containing nine bonded groups derived from the reactant compounds.

The term "metathesis decamer" refers to the product of one or more metathesis reactions wherein ten molecules of two or more reactant compounds, which can be the same or different and each with one or more carbon-carbon double bonds, are bonded together via one or more of the carbon-carbon double bonds in each of the reactant compounds as a result of the one or more metathesis reactions, the decamer containing ten bonded groups derived from the reactant compounds.

The term "metathesis oligomer" refers to the product of one or more metathesis reactions wherein two or more molecules (e.g., 2 to about 10, or 2 to about 4) of two or more reactant compounds, which can be the same or different and each with one or more carbon-carbon double bonds, are bonded together via one or more of the carbon-carbon double bonds in each of the reactant compounds as a result of the one or more metathesis reactions, the oligomer containing a few (e.g., 2 to about 10, or 2 to about 4) bonded groups derived from the reactant compounds. In some embodiments, the term "metathesis oligomer" may include metathesis reactions wherein greater than ten molecules of two or more reactant compounds, which can be the same or different and each with one or more carbon-carbon double bonds, are bonded together via one or more of the carbon-carbon double bonds in each of the reactant compounds as a result of the one or more metathesis reactions, the oligomer containing greater than ten bonded groups derived from the reactant compounds.

As used herein, the terms "metathesize" and "metathesizing" may refer to the reacting of a unsaturated polyol ester feedstock in the presence of a metathesis catalyst to form a metathesized unsaturated polyol ester product comprising a new olefinic compound and/or esters. Metathesizing may refer to cross-metathesis (a.k.a. co-metathesis), self-metathesis, ring-opening metathesis, ring-opening metathesis polymerizations ("ROMP"), ring-closing metathesis ("RCM"), and acyclic diene metathesis ("ADMET"). As a non-limiting example, metathesizing may refer to reacting two triglycerides present in a natural feedstock (self-metathesis) in the presence of a metathesis catalyst, wherein each triglyceride has an unsaturated carbon-carbon double bond, thereby forming an oligomer having a new mixture of olefins and esters that may comprise one or more of: metathesis monomers, metathesis dimers, metathesis trimers, metathesis tetramers, metathesis pentamers, and higher order metathesis oligomers (e.g., metathesis hexamers, metathesis, metathesis heptamers, metathesis octamers, metathesis nonamers, metathesis decamers, and higher than metathesis decamers and above).

As used herein, the term "polyol" means an organic material comprising at least two hydroxy moieties.

As used herein, the articles including "a" and "an" when used in a claim, are understood to mean one or more of what is claimed or described.

As used herein, the terms "include", "includes" and "including" are meant to be non-limiting.

Unless otherwise noted, all component or composition levels are in reference to the active portion of that component or composition, and are exclusive of impurities, for example, residual solvents or by-products, which may be present in commercially available sources of such components or compositions.

All percentages and ratios are calculated by weight unless otherwise indicated. All percentages and ratios are calculated based on the total composition unless otherwise indicated.

It should be understood that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

### Compositions, Articles and Methods of Use

**Table 1 Compositions suitable for incorporation in a lubricating member of the invention, the invention being in accordance with the claims.**

| Comp. No. | | Composition | |
|---|---|---|---|
| 1 | | A composition comprising, | |
| | | a) | a metathesized unsaturated polyol ester, said metathesized unsaturated polyol ester having one or more of the following properties: |
| | | | (i) a weight average molecular weight of from about 5,000 Daltons to about 50,000 Daltons, from about 5,500 Daltons to about 50,000 Daltons, from about 5,500 Daltons to about 40,000 Daltons, or from about 6,000 Daltons to about 30,000 Daltons; |
| | | | (ii) an oligomer index from greater than 0 to 1, from 0.001 to 1, 0.01 to 1, or from 0.05 to 1; |
| | | | (iii) an iodine value of from about 30 to about 200, from about 30 to about 150, from about 30 to about 120, or from about 50 to about 110. |
| 2 | In one aspect of said composition 1 of Table 1, said metathesized unsaturated polyol ester has the weight average molecular weight property from a)(i) above. | | |
| 3 | In one aspect of said composition 1 of Table 1, said metathesized unsaturated polyol ester has the oligomer index property from a)(ii) above. | | |
| 4 | In one aspect of said composition 1 of Table 1, said metathesized unsaturated polyol ester has the iodine value property from a)(iii) above. | | |
| 5 | In one aspect of said composition 1 of Table 1, said metathesized unsaturated polyol ester has the property from a)(i) and from a)(ii) above. | | |
| 6 | In one aspect of said composition 1 of Table 1, said metathesized unsaturated polyol ester has the properties from a)(i) and from a)(iii) above. | | |
| 7 | In one aspect of said composition 1 of Table 1, said metathesized unsaturated polyol ester has the properties from a)(ii) and from a)(iii) above. | | |
| 8 | In one aspect of said composition 1 of Table 1, said metathesized unsaturated polyol ester has the properties from a)(i), a)(ii) and from a)(iii) above. | | |
| 9 | In one aspect, of compositions 1, 2, 3, 4, 5, 6, 7, and 8 of Table 1, said metathesized unsaturated polyol ester has a free hydrocarbon content, based on total weight of metathesized unsaturated polyol ester, of from about 0% to about 5%, from about 0.1% to about 5%, from about 0.1% to about 4%, or from about 0.1 to about 3%. | | |
| 10 | In one aspect of Table 1 Compositions 1, 2, 3, 4, 5, 6, 7, 8, and 9 the metathesized unsaturated polyol ester is metathesized at least once. | | |
| 11 | In one aspect, of compositions 1, 2, 3, 4, 5, 6, 7, 8, 9 and 10 of Table 1, said composition comprises, based on total composition weight, from about 0.1% to about 50%, from about 0.5% to about 30%, or from about 1% to about 20% of said metathesized unsaturated polyol ester. | | |

In one aspect, Table 1 Compositions 1, 2, 3, 4, 5, 6, 7, 8, 9, 10 and 11; the metathesized unsaturated polyol ester is derived from a natural polyol ester and/or a synthetic polyol ester, in one aspect, said natural polyol ester is selected from the group consisting of a vegetable oil, an animal fat, an algae oil and mixtures thereof; and said synthetic polyol ester is derived from a material selected from the group consisting of ethylene glycol, propylene glycol, glycerol, polyglycerol, polyethylene glycol, polypropylene glycol, poly(tetramethylene ether) glycol, pentaerythritol, dipentaerythritol, tripentaerythritol, trimethylolpropane, neopentyl glycol, a sugar, in one aspect, sucrose, and mixtures thereof.

In one aspect, Table 1 Compositions 1, 2, 3, 4, 5, 6, 7, 8, 9, 10 and 11; the metathesized unsaturated polyol ester is selected from the group consisting of metathesized Abyssinian oil, metathesized Almond Oil, metathesized Apricot Oil, metathesized Apricot Kernel oil, metathesized Argan oil, metathesized Avocado Oil, metathesized Babassu Oil, metathesized Baobab Oil, metathesized Black Cumin Oil, metathesized Black Currant Oil, metathesized Borage Oil, metathesized Camelina oil, metathesized Carinata oil, metathesized Canola oil, metathesized Castor oil, metathesized Cherry Kernel Oil, metathesized Coconut oil, metathesized Corn oil, metathesized Cottonseed oil, metathesized Echium Oil, metathesized Evening Primrose Oil, metathesized Flax Seed Oil, metathesized Grape Seed Oil, metathesized Grapefruit Seed Oil, metathesized Hazelnut Oil, metathesized Hemp Seed Oil, metathesized Jatropha oil, metathesized Jojoba Oil, metathesized Kukui Nut Oil, metathesized Linseed Oil, metathesized Macadamia Nut Oil, metathesized Meadowfoam Seed Oil, metathesized Moringa Oil, metathesized Neem Oil, metathesized Olive Oil, metathesized Palm Oil, metathesized Palm Kernel Oil, metathesized Peach Kernel Oil, metathesized Peanut Oil, metathesized Pecan Oil, metathesized Pennycress oil, metathesized Perilla Seed Oil, metathesized Pistachio Oil, metathesized Pomegranate Seed Oil, metathesized Pongamia oil, metathesized Pumpkin Seed Oil, metathesized Raspberry Oil, metathesized Red Palm Olein, metathesized Rice Bran Oil, metathesized Rosehip Oil, metathesized Safflower Oil, metathesized Seabuckthorn Fruit Oil, metathesized Sesame Seed Oil, metathesized Shea Olein, metathesized Sunflower Oil, metathesized Soybean Oil, metathesized Tonka Bean Oil, metathesized Tung Oil, metathesized Walnut Oil, metathesized Wheat Germ Oil, metathesized High Oleoyl Soybean Oil, metathesized High Oleoyl Sunflower Oil, metathesized High Oleoyl Safflower Oil, metathesized High Erucic Acid Rapeseed Oil, and mixtures thereof.

### Methods of Making Compositions

The compositions of the present invention can be formulated into any suitable form and prepared by any process chosen by the formulator, non-limiting examples of which are described in U.S. 5,879,584 nce. For example, the metathesized unsaturated polyol esters can be combined directly with the composition's other ingredients without pre-emulsification and/or pre-mixing to form the finished products. Alternatively, the metathesized unsaturated polyol esters can be combined with surfactants or emulsifiers, solvents, suitable adjuncts, and/or any other suitable ingredients to prepare emulsions prior to compounding the finished products.

Suitable equipment for use in the processes disclosed herein may include continuous stirred tank reactors, homogenizers, turbine agitators, recirculating pumps, paddle mixers, plough shear mixers, ribbon blenders, vertical axis granulators and drum mixers, both in batch and, where available, in continuous process configurations, spray dryers, and extruders. Such equipment can be obtained from Lodige GmbH (Paderborn, Germany), Littleford Day, Inc. (Florence, Kentucky, U.S.A.), Forberg AS (Larvik, Norway), Glatt Ingenieurtechnik GmbH (Weimar, Germany), Niro (Soeborg, Denmark), Hosokawa Bepex Corp. (Minneapolis, Minnesota, U.S.A.), Arde Barinco (New Jersey, U.S.A.).

### Lipid phase

According to the invention the lubricating member is a solid lubricating member at 25°C and may comprise from about 20% to about 90% by weight, preferably from about 20% to about 80% by weight of a lipid phase. The lipid phase comprises a lipophilic structurant and a liquid phase contained within the lipophilic structurant. The lipid phase comprises from about 10% to about 70%, preferably from about 10% to 60%, more preferably from about 20% to about 40%, even more preferably from about 25% to about 35% by weight of the lubricating member of a lipophilic structurant.

The melting point of the lipophilic structurant is greater than 45°C to less than 60°C and is thus preferably a solid at 25°C. The melting point is determined according to ASTM D5440-93. If the lipophilic structurant comprises more than one material, the melting point is determined for the resultant mixture as described hereinafter. In one embodiment the lipophilic structurant has a melting point of from about 45°C to about 5°C less than the melting point of said water soluble polymer. The lipophilic structurant is preferably water insoluble. It has been surprisingly found that by providing a lipohilic structurant having a melting point below 60°C enables both the ready addition of liquid phase components and also water soluble polymers, which are immiscible therein without melting of the water soluble polymer during manufacture. The later thereby avoids thermal degradation and thereby the lipophilic structurant provides a solid chassis at room temperature (25°C) to contain the ingredient components which also deliver lubrication to the skin and other benefit agents during the shaving process. Moreover, the lipophilic structurant also enhances skin feel benefits.

Suitable lipophilic structurants for use herein include C14 or greater, preferably C14 to C20, more preferably C16 to C18 chain length fatty acyls such as fatty acids, fatty alcohols and esters, triglycerides, waxes and mixtures thereof. Particularly preferred are C14-C20 alcohols, in particular cetyl and stearyl alcohols and mixtures thereof.

Suitable lipophilic structurants also include natural, synthetic and silicone waxes. As used herein, the term "wax" includes, but is not limited to, any material that is solid at 45°C, preferably at 25°C; and are very slightly soluble in water, preferably practically insoluble in water according to the United States' Pharmacopeia (USP) definition in 31/NF 26 Vol. 2 General Notices, Page Xvii. According to that definition, this means that 1000 to 10000 parts of water are needed to dissolve 1 part solute and that more than 10,000 parts of water are needed to dissolve 1 part solute respectively.

The lipophilic structurant and or lubricating member preferably comprises less than 5%, preferably less than 1% by weight and more preferably is substantially free of lathering soap (i.e. salts of fatty acids such as C4-30 carboxylic acids) or lathering surfactant. A lathering surfactant is defined as a surfactant which when combined with water and mechanically agitated generate a foam or later. Lathering surfactants include anionic and amphoteric lathering surfactants and mixtures thereof. Anionic lathering surfactants include sarcosinates, sulfates, sulfonate, isethionate, taurates, phosphates, lactylates, glutamates, alkali metal salts of fatty acids (i.e. soaps) having from 8 to 24 carbons, and mixtures thereof.

The wax may comprise natural wax, synthetic wax or mixtures thereof. Natural waxes may be plant, animal or mineral derived. Non-limiting examples of suitable natural waxes include Beeswax, Copernicia Cerifera (Carnauba) Wax, Euphorbia Cerifera (Candelilla) Wax, Jojoba Wax, Oryza Sativa (Rice) Bran Wax, Lemon peel wax, Soybean wax, Sunflower wax and mixtures thereof.

Non-limiting examples of suitable synthetic waxes include Hydrogenated Jojoba Wax, synthetic and siliconyl jojoba wax, Hydrogenated Microcrystalline Wax, Microcrystalline Wax, synthetic, siliconyl and Hydrogenated Rice Bran Wax, Ceresin, Ozokerite, Paraffin, benhenyl beeswax, synthetic, siliconyl and hydrogenated Beeswax, synthetic, hydrogenated and siliconyl Candelilla Wax, synthetic, hydrogenated and siliconyl Carnauba, wax, , synthetic, hydrogenated and siliconyl lemon peel wax, synthetic, siliconyl and hydrogenated soybean wax, synthetic, siliconyl and hydrogenated sunflower wax and mixtures thereof. Preferred natural and synthetic waxes are Beeswax, Microcrystalline wax, Candellila wax, Ozokerite, and mixtures thereof.

Non-limiting examples of suitable silicone waxes include Stearyoxy trimethylsilane such as DC580 wax, C30-45 alkyl methicone available as DC AMS-C30 Cosmetic Wax, stearyoxymethyl silane available as DC Silkywax 10, C24-54 alkyl methicone such as DC ST-Wax 30, C30-45 Alkyldimethylsilyl, Polypropyl-silsesquioxane, available as DC SW-8005 resin wax, and mixtures thereof.

Particularly preferred lipophilic structurants may be selected from cetyl alcohol, stearyl alcohol, microcrystalline wax, stearyloxy trimethylsilane and mixtures thereof.

### Liquid phase

The lubricating member may further comprise from about 10% to about 70%, preferably from about 10% to about 60%, more preferably from about 10% to about 40%, by weight of the lubricating member of a liquid phase. In one embodiment the liquid phase comprises a hydrophobic material or mixtures thereof. The liquid phase may provide a number of in use benefits such as lubrication, skin feel and cooling sensation. The liquid phase is contained within the solid lubricating member by the lipophilic structurant.

The liquid phase has a melting point of 45°C or less, preferably 40°C or less, even more preferably 30°C or less, most preferably 25°C or less. The melting point is determined according to ASTM D5440-93. Preferably the liquid phase and the hydrophobic material is liquid at 25°C. The use of a liquid phase enables the materials to be readily added to the lipophilic structurant upon melting thereof. In another preferred embodiment the liquid phase hydrophobic material or mixtures thereof may be very slightly soluble and have a melting point of 45°C or less as defined herein above and be miscible with oneanother. In another embodiment the melting point of the mixture of liquid phase and the lipophilic structurant is preferably from 45°C to 5°C less than the melting point of the water soluble polymer.

The liquid phase comprises a metathesized unsaturated polyol ester.

### Metathesized unsaturated polyol ester

Exemplary metathesized unsaturated polyol esters and their starting materials are set forth in U.S. Patent Applications U.S. 2009/0220443 A1, U.S. 2013/0344012 A1 and US 2014/0357714 A1 A metathesized unsaturated polyol ester refers to the product obtained when one or more unsaturated polyol ester ingredient(s) are subjected to a metathesis reaction. Metathesis is a catalytic reaction that involves the interchange of alkylidene units among compounds containing one or more double bonds (i.e., olefinic compounds) via the formation and cleavage of the carbon-carbon double bonds. Metathesis may occur between two of the same molecules (often referred to as self-metathesis) and/or it may occur between two different molecules (often referred to as cross-metathesis). Self-metathesis may be represented schematically as shown in Equation I.

R¹-CH=CH-R²+R¹-CH=CH-R² H R¹-CH=CH-R¹+R²-CH=CH-R² (1)

where R¹ and R² are organic groups.

Cross-metathesis may be represented schematically as shown in Equation II.

**R¹-CH-CH-R² + R³-CH-CH-R⁴ ↔ R¹-CH-CH-R³ + R¹-CH-CH-R⁴ + R²-CH=CH-R³ + R²-CH-CH-R⁴ + R¹-CH-CH-R¹ + R²-CH=CH-R² + R³-CH-CH-R³ + R⁴-CH-CH-R⁴** **(II)**

where R¹, R², R³, and R⁴ are organic groups.

When a polyol ester comprises molecules having more than one carbon-carbon double bond, self-metathesis may result in oligomerization or polymerization of the unsaturates in the starting material. For example, Equation C depicts metathesis oligomerization of a representative species (e.g., a polyol ester) having more than one carbon-carbon double bond. In Equation C, the self-metathesis reaction results in the formation of metathesis dimers, metathesis trimers, and metathesis tetramers. Although not shown, higher order oligomers such as metathesis pentamers, hexamers, heptamers, octamers, nonamers, decamers, and higher than decamers, and mixtures of two or more thereof, may also be formed. The number of metathesis repeating units or groups in the metathesized natural oil may range from 1 to about 100, or from 2 to about 50, or from 2 to about 30, or from 2 to about 10, or from 2 to about 4. The molecular weight of the metathesis dimer may be greater than the molecular weight of the unsaturated polyol ester from which the dimer is formed. Each of the bonded polyol ester molecules may be referred to as a "repeating unit or group." Typically, a metathesis trimer may be formed by the cross-metathesis of a metathesis dimer with an unsaturated polyol ester. Typically, a metathesis tetramer may be formed by the cross-metathesis of a metathesis trimer with an unsaturated polyol ester or formed by the cross-metathesis of two metathesis dimers.

Equation C R¹-HC=CH-R²-HC=CH-R³ + R¹HC=CH-R²-HC=CH-R³ ↔ R¹-HC=CH-R²-HC=CH-R²-HC=CH-R³ + (other products) (metathesis dimer) R¹-R²-HC=CH-R²-HC=CH-R³ + R¹-HC=CH-R²-HC=CH-R³ ↔ R¹-HC=CH-R²-HC=CH-R²-HC=CH-R²-HC=CH-R³ + (other products) (metathesis trimer) R¹-HC=CH-R²-HC=CH-R²-HC=CH-R²-HC=CH-R³ + R¹-HC=CH-R²-HC=CH-R³ ↔ R¹-HC=CH-R²-HC=CH-R²-HC=CH-R²-HC=CH-R²-HC=CH-R³ + (other products) (metathesis tetramer)

where R¹, R², and R³ are organic groups.

As a starting material, metathesized unsaturated polyol esters are prepared from one or more unsaturated polyol esters. As used herein, the term "unsaturated polyol ester" refers to a compound having two or more hydroxyl groups wherein at least one of the hydroxyl groups is in the form of an ester and wherein the ester has an organic group including at least one carbon-carbon double bond. In many embodiments, the unsaturated polyol ester can be represented by the general structure (I): 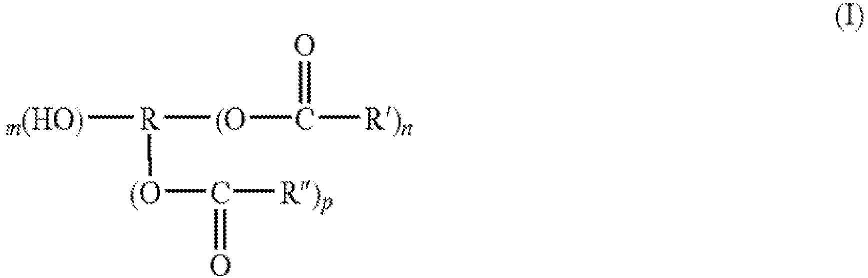
where n≥1;
m≥0;
p≥0;
(n+m+p) ≥2;
R is an organic group;
R' is an organic group having at least one carbon-carbon double bond; and
R" is a saturated organic group.

In many embodiments of the invention, the unsaturated polyol ester is an unsaturated polyol ester of glycerol. Unsaturated polyol esters of glycerol have the general structure (II): 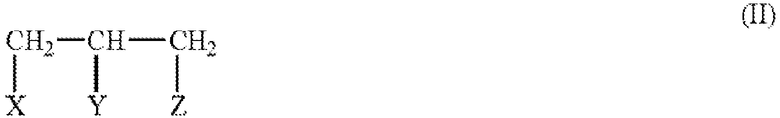 where -X, -Y, and -Z are independently selected from the group consisting of:

-OH; -(O-C(=O)-R'); and -(O-C(=O)- R");

where -R' is an organic group having at least one carbon-carbon double bond and -R" is a saturated organic group.

In structure (II), at least one of -X, -Y, and -Z is -(O-C(=O)-R').

In some embodiments, R' is a straight or branched chain hydrocarbon having about 50 or less carbon atoms (e.g., about 36 or less carbon atoms or about 26 or less carbon atoms) and at least one carbon-carbon double bond in its chain. In some embodiments, R' is a straight or branched chain hydrocarbon having about 6 carbon atoms or greater (e.g., about 10 carbon atoms or greater or about 12 carbon atoms or greater) and at least one carbon-carbon double bond in its chain. In some embodiments, R' may have two or more carbon-carbon double bonds in its chain. In other embodiments, R' may have three or more double bonds in its chain. In exemplary embodiments, R' has 17 carbon atoms and 1 to 3 carbon-carbon double bonds in its chain. Representative examples of R' include:

-(CH₂)₇CH=CH-(CH₂)₇-CH₃;

-(CH₂)₇CH=CH-CH₂-CH=CH-(CH₂)₄- CH₃;

and

-(CH₂)₇CH=CH-CH₂-CH=CH-CH₂- CH=CH-CH₂-CH₃.

In some embodiments, R" is a saturated straight or branched chain hydrocarbon having about 50 or less carbon atoms (e.g., about 36 or less carbon atoms or about 26 or less carbon atoms). In some embodiments, R" is a saturated straight or branched chain hydrocarbon having about 6 carbon atoms or greater (e.g., about 10 carbon atoms or greater or about 12 carbon atoms or greater. In exemplary embodiments, R" has 15 carbon atoms or 17 carbon atoms.

Sources of unsaturated polyol esters of glycerol include synthesized oils, natural oils (e.g., vegetable oils, algae oils, bacterial derived oils, and animal fats), combinations of these, and the like. Recycled used vegetable oils may also be used. Representative non-limiting examples of vegetable oils include Abyssinian oil, Almond Oil, Apricot Oil, Apricot Kernel oil, Argan oil, Avocado Oil, Babassu Oil, Baobab Oil, Black Cumin Oil, Black Currant Oil, Borage Oil, Camelina oil, Carinata oil, Canola oil, Castor oil, Cherry Kernel Oil, Coconut oil, Corn oil, Cottonseed oil, Echium Oil, Evening Primrose Oil, Flax Seed Oil, Grape Seed Oil, Grapefruit Seed Oil, Hazelnut Oil, Hemp Seed Oil, Jatropha oil, Jojoba Oil, Kukui Nut Oil, Linseed Oil, Macadamia Nut Oil, Meadowfoam Seed Oil, Moringa Oil, Neem Oil, Olive Oil, Palm Oil, Palm Kernel Oil, Peach Kernel Oil, Peanut Oil, Pecan Oil, Pennycress oil, Perilla Seed Oil, Pistachio Oil, Pomegranate Seed Oil, Pongamia oil, Pumpkin Seed Oil, Raspberry Oil, Red Palm Olein, Rice Bran Oil, Rosehip Oil, Safflower Oil, Seabuckthorn Fruit Oil, Sesame Seed Oil, Shea Olein, Sunflower Oil, Soybean Oil, Tonka Bean Oil, Tung Oil, Walnut Oil, Wheat Germ Oil, High Oleoyl Soybean Oil, High Oleoyl Sunflower Oil, High Oleoyl Safflower Oil, High Erucic Acid Rapeseed Oil, combinations of these, and the like. Representative non-limiting examples of animal fats include lard, tallow, chicken fat, yellow grease, fish oil, emu oil, combinations of these, and the like. A representative non-limiting example of a synthesized oil includes tall oil, which is a byproduct of wood pulp manufacture. In some embodiments, the natural oil is refined, bleached, and/or deodorized.

Other examples of unsaturated polyol esters include esters such as those derived from ethylene glycol or propylene glycol, polyethylene glycol, polypropylene glycol, or poly(tetramethylene ether) glycol, esters such as those derived from pentaerythritol, dipentaerythritol, tripentaerythritol, trimethylolpropane, or neopentyl glycol, or sugar esters such as SEFOSE^{®}. Sugar esters such as SEFOSE^{®} include one or more types of sucrose polyesters, with up to eight ester groups that could undergo a metathesis exchange reaction. Sucrose polyesters are derived from a natural resource and therefore, the use of sucrose polyesters can result in a positive environmental impact. Sucrose polyesters are polyester materials, having multiple substitution positions around the sucrose backbone coupled with the chain length, saturation, and derivation variables of the fatty chains. Such sucrose polyesters can have an esterification ("IBAR") of greater than about 5. In one embodiment the sucrose polyester may have an IBAR of from about 5 to about 8. In another embodiment the sucrose polyester has an IBAR of about 5-7, and in another embodiment the sucrose polyester has an IBAR of about 6. In yet another embodiment the sucrose polyester has an IBAR of about 8. As sucrose polyesters are derived from a natural resource, a distribution in the IBAR and chain length may exist. For example a sucrose polyester having an IBAR of 6, may contain a mixture of mostly IBAR of about 6, with some IBAR of about 5 and some IBAR of about 7. Additionally, such sucrose polyesters may have a saturation or iodine value ("IV") of about 3 to about 140. In another embodiment the sucrose polyester may have an IV of about 10 to about 120. In yet another embodiment the sucrose polyester may have an IV of about 20 to 100. Further, such sucrose polyesters have a chain length of about C₁₂ to C₂₀ but are not limited to these chain lengths.

Non-limiting examples of sucrose polyesters suitable for use include SEFOSE^{®} 1618S, SEFOSE^{®} 1618U, SEFOSE^{®} 1618H, Sefa Soyate IMF 40, Sefa Soyate LP426, SEFOSE^{®} 2275, SEFOSE^{®} C1695, SEFOSE^{®} C18:0 95, SEFOSE^{®} C1495, SEFOSE^{®} 1618H B6, SEFOSE^{®} 1618S B6, SEFOSE^{®} 1618U B6, Sefa Cottonate, SEFOSE^{®} C1295, Sefa C895, Sefa C1095, SEFOSE^{®} 1618S B4.5, all available from The Procter and Gamble Co. of Cincinnati, Ohio.

Other examples of suitable polyol esters may include but not be limited to sorbitol esters, maltitol esters, sorbitan esters, maltodextrin derived esters, xylitol esters, polyglycerol esters, and other sugar derived esters.

Natural oils of the type described herein typically are composed of triglycerides of fatty acids. These fatty acids may be either saturated, monounsaturated or polyunsaturated and contain varying chain lengths ranging from C₈ to C₃₀. The most common fatty acids include saturated fatty acids such as lauric acid (dodecanoic acid), myristic acid (tetradecanoic acid), palmitic acid (hexadecanoic acid), stearic acid (octadecanoic acid), arachidic acid (eicosanoic acid), and lignoceric acid (tetracosanoic acid); unsaturated acids include such fatty acids as palmitoleic (a C₁₆ acid), and oleic acid (a C₁₈ acid); polyunsaturated acids include such fatty acids as linoleic acid (a di-unsaturated C₁₈ acid), linolenic acid (a tri-unsaturated C₁₈ acid), and arachidonic acid (a tetra-unsubstituted C₂₀ acid). The natural oils are further comprised of esters of these fatty acids in random placement onto the three sites of the trifunctional glycerine molecule. Different natural oils will have different ratios of these fatty acids, and within a given natural oil there is a range of these acids as well depending on such factors as where a vegetable or crop is grown, maturity of the vegetable or crop, the weather during the growing season, etc. Thus, it is difficult to have a specific or unique structure for any given natural oil, but rather a structure is typically based on some statistical average. For example soybean oil contains a mixture of stearic acid, oleic acid, linoleic acid, and linolenic acid in the ratio of 15:24:50: 11, and an average number of double bonds of 4.4-4.7 per triglyceride. One method of quantifying the number of double bonds is the iodine value (IV) which is defined as the number of grams of iodine that will react with 100 grams of oil. Therefore for soybean oil, the average iodine value range is from 120-140. Soybean oil may comprise about 95% by weight or greater (e.g., 99% weight or greater) triglycerides of fatty acids. Major fatty acids in the polyol esters of soybean oil include saturated fatty acids, as a non-limiting example, palmitic acid (hexadecanoic acid) and stearic acid (octadecanoic acid), and unsaturated fatty acids, as a non-limiting example, oleic acid (9-octadecenoic acid), linoleic acid (9,12octadecadienoic acid), and linolenic acid (9,12,15-octadecatrienoic acid).

In an exemplary embodiment, the vegetable oil is canola oil, for example, refined, bleached, and deodorized canola oil (i.e., RBD canola oil). Canola oil is an unsaturated polyol ester of glycerol that typically comprises about 95% weight or greater (e.g., 99% weight or greater) triglycerides of fatty acids. Major fatty acids in the polyol esters of canola oil include saturated fatty acids, for example, palmitic acid (hexadecanoic acid) and stearic acid (octadecanoic acid), and unsaturated fatty acids, for example, oleic acid (9-octadecenoic acid), linoleic acid (9,12-octadecadienoic acid), and linolenic acid (9,12,15-octadecatrienoic acid). Canola oil is a highly unsaturated vegetable oil with many of the triglyceride molecules having at least two unsaturated fatty acids (i.e., a polyunsaturated triglyceride).

In exemplary embodiments, an unsaturated polyol ester is self-metathesized in the presence of a metathesis catalyst to form a metathesized composition. Typically, after metathesis has occurred, the metathesis catalyst is removed from the resulting product. One method of removing the catalyst is treatment of the metathesized product with clay. In many embodiments, the metathesized composition comprises one or more of: metathesis monomers, metathesis dimers, metathesis trimers, metathesis tetramers, metathesis pentamers, and higher order metathesis oligomers (e.g., metathesis hexamers). A metathesis dimer refers to a compound formed when two unsaturated polyol ester molecules are covalently bonded to one another by a self-metathesis reaction. In many embodiments, the molecular weight of the metathesis dimer is greater than the molecular weight of the individual unsaturated polyol ester molecules from which the dimer is formed. A metathesis trimer refers to a compound formed when three unsaturated polyol ester molecules are covalently bonded together by metathesis reactions. In many embodiments, a metathesis trimer is formed by the cross-metathesis of a metathesis dimer with an unsaturated polyol ester. A metathesis tetramer refers to a compound formed when four unsaturated polyol ester molecules are covalently bonded together by metathesis reactions. In many embodiments, a metathesis tetramer is formed by the cross-metathesis of a metathesis trimer with an unsaturated polyol ester. Metathesis tetramers may also be formed, for example, by the cross-metathesis of two metathesis dimers. Higher order metathesis products may also be formed. For example, metathesis pentamers and metathesis hexamers may also be formed. The self-metathesis reaction also results in the formation of internal olefin compounds that may be linear or cyclic. If the metathesized polyol ester is fully or partially hydrogenated, the linear and cyclic olefins would typically be fully or partially converted to the corresponding saturated linear and cyclic hydrocarbons. The linear/cyclic olefins and saturated linear/cyclic hydrocarbons may remain in the metathesized polyol ester or they may be removed or partially removed from the metathesized polyol ester using one or more known stripping techniques, including but not limited to wipe film evaporation, falling film evaporation, rotary evaporation, steam stripping, vacuum distillation, etc.

In some embodiments, the unsaturated polyol ester is partially hydrogenated before being metathesized. For example, in some embodiments, the unsaturated polyol ester is partially hydrogenated to achieve an iodine value (IV) of about 120 or less before subjecting the partially hydrogenated polyol ester to metathesis.

In some embodiments, the unsaturated polyol ester may be hydrogenated (e.g., fully or partially hydrogenated) in order to improve the stability of the oil or to modify its viscosity or other properties. Representative techniques for hydrogenating unsaturated polyol esters are known in the art and are discussed herein.

In some embodiments, the natural oil is winterized. Winterization refers to the process of: (1) removing waxes and other non-triglyceride constituents, (2) removing naturally occurring high-melting triglycerides, and (3) removing high-melting triglycerides formed during partial hydrogenation. Winterization may be accomplished by known methods including, for example, cooling the oil at a controlled rate in order to cause crystallization of the higher melting components that are to be removed from the oil. The crystallized high melting components are then removed from the oil by filtration resulting in winterized oil. Winterized soybean oil is commercially available from Cargill, Incorporated (Minneapolis, Minn.).

In other embodiments, the metathesized unsaturated polyol esters can be used as a blend with one or more fabric care benefit agents and/or fabric softening actives.

### Method of Making Metathesized Unsaturated Polyol Ester

The self-metathesis of unsaturated polyol esters is typically conducted in the presence of a catalytically effective amount of a metathesis catalyst. The term "metathesis catalyst" includes any catalyst or catalyst system that catalyzes a metathesis reaction. Any known or future-developed metathesis catalyst may be used, alone or in combination with one or more additional catalysts. Suitable homogeneous metathesis catalysts include combinations of a transition metal halide or oxo-halide (e.g., WOCl₄ or WCl₆) with an alkylating cocatalyst (e.g., Me₄Sn), or alkylidene (or carbene) complexes of transition metals, particularly Ru or W. These include first and second-generation Grubbs catalysts, Grubbs-Hoveyda catalysts, and the like. Suitable alkylidene catalysts have the general structure:

M[X¹X²L¹L²(L³)*ₙ*]=*Cₘ*=C(R¹)R²

Where M is a Group 8 transition metal, L¹, L², and L³ are neutral electron donor ligands, n is 0 (such that L³ may not be present) or 1, m is 0,1, or 2, X¹ and X² are anionic ligands, and R¹ and R² are independently selected from H, hydrocarbyl, substituted hydrocarbyl, heteroatom-containing hydrocarbyl, substituted heteroatom-containing hydrocarbyl, and functional groups. Any two or more of X¹, X², L¹, L², L³, R¹ and R² can form a cyclic group and any one of those groups can be attached to a support.

First-generation Grubbs catalysts fall into this category where m=n=0 and particular selections are made for n, X¹, X², L¹, L², L³, R¹ and R² as described in U.S. Pat. Appl. Publ. No. 2010/0145086, the teachings of which related to all metathesis catalysts are incorporated herein by reference.

Second-generation Grubbs catalysts also have the general formula described above, but L¹ is a carbene ligand where the carbene carbon is flanked by N, O, S, or P atoms, preferably by two N atoms. Usually, the carbene ligand is part of a cyclic group. Examples of suitable second-generation Grubbs catalysts also appear in the '086 publication.

In another class of suitable alkylidene catalysts, L¹ is a strongly coordinating neutral electron donor as in first-and second-generation Grubbs catalysts, and L² and L³ are weakly coordinating neutral electron donor ligands in the form of optionally substituted heterocyclic groups. Thus, L² and L³ are pyridine, pyrimidine, pyrrole, quinoline, thiophene, or the like.

In yet another class of suitable alkylidene catalysts, a pair of substituents is used to form a bi- or tridentate ligand, such as a biphosphine, dialkoxide, or alkyldiketonate. Grubbs-Hoveyda catalysts are a subset of this type of catalyst in which L² and R² are linked. Typically, a neutral oxygen or nitrogen coordinates to the metal while also being bonded to a carbon that is α-, β-, or γ- with respect to the carbene carbon to provide the bidentate ligand. Examples of suitable Grubbs-Hoveyda catalysts appear in the '086 publication.

The structures below provide just a few illustrations of suitable catalysts that may be used:

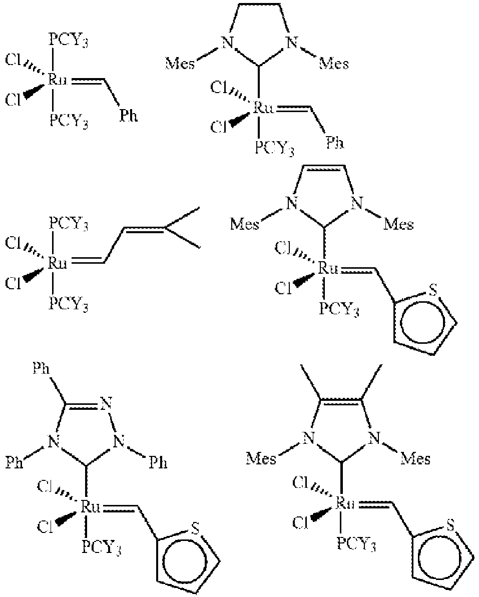

An immobilized catalyst can be used for the metathesis process. An immobilized catalyst is a system comprising a catalyst and a support, the catalyst associated with the support. Exemplary associations between the catalyst and the support may occur by way of chemical bonds or weak interactions (e.g. hydrogen bonds, donor acceptor interactions) between the catalyst, or any portions thereof, and the support or any portions thereof. Support is intended to include any material suitable to support the catalyst. Typically, immobilized catalysts are solid phase catalysts that act on liquid or gas phase reactants and products. Exemplary supports are polymers, silica or alumina. Such an immobilized catalyst may be used in a flow process. An immobilized catalyst can simplify purification of products and recovery of the catalyst so that recycling the catalyst may be more convenient.

In certain embodiments, prior to the metathesis reaction, the unsaturated polyol ester feedstock may be treated to render the natural oil more suitable for the subsequent metathesis reaction. In one embodiment, the treatment of the unsaturated polyol ester involves the removal of catalyst poisons, such as peroxides, which may potentially diminish the activity of the metathesis catalyst. Non-limiting examples of unsaturated polyol ester feedstock treatment methods to diminish catalyst poisons include those described in PCT/US2008/09604, PCT/US2008/09635, and U.S. patent application Ser. Nos. 12/672,651 and 12/672,652 In certain embodiments, the unsaturated polyol ester feedstock is thermally treated by heating the feedstock to a temperature greater than 100° C. in the absence of oxygen and held at the temperature for a time sufficient to diminish catalyst poisons in the feedstock. In other embodiments, the temperature is between approximately 100° C. and 300° C., between approximately 120° C. and 250° C., between approximately 150° C. and 210° C., or approximately between 190 and 200° C. In one embodiment, the absence of oxygen is achieved by sparging the unsaturated polyol ester feedstock with nitrogen, wherein the nitrogen gas is pumped into the feedstock treatment vessel at a pressure of approximately 10 atm (150 psig).

In certain embodiments, the unsaturated polyol ester feedstock is chemically treated under conditions sufficient to diminish the catalyst poisons in the feedstock through a chemical reaction of the catalyst poisons. In certain embodiments, the feedstock is treated with a reducing agent or a cation-inorganic base composition. Non-limiting examples of reducing agents include bisulfate, borohydride, phosphine, thiosulfate, and combinations thereof.

In certain embodiments, the unsaturated polyol ester feedstock is treated with an adsorbent to remove catalyst poisons. In one embodiment, the feedstock is treated with a combination of thermal and adsorbent methods. In another embodiment, the feedstock is treated with a combination of chemical and adsorbent methods. In another embodiment, the treatment involves a partial hydrogenation treatment to modify the unsaturated polyol ester feedstock's reactivity with the metathesis catalyst. Additional non-limiting examples of feedstock treatment are also described below when discussing the various metathesis catalysts.

In certain embodiments, a ligand may be added to the metathesis reaction mixture. In many embodiments using a ligand, the ligand is selected to be a molecule that stabilizes the catalyst, and may thus provide an increased turnover number for the catalyst. In some cases the ligand can alter reaction selectivity and product distribution. Examples of ligands that can be used include Lewis base ligands, such as, without limitation, trialkylphosphines, for example tricyclohexylphosphine and tributyl phosphine; triarylphosphines, such as triphenylphosphine; diarylalkylphosphines, such as, diphenylcyclohexylphosphine; pyridines, such as 2,6-dimethylpyridine, 2,4,6-trimethylpyridine; as well as other Lewis basic ligands, such as phosphine oxides and phosphinites. Additives may also be present during metathesis that increase catalyst lifetime.

Any useful amount of the selected metathesis catalyst can be used in the process. For example, the molar ratio of the unsaturated polyol ester to catalyst may range from about 5:1 to about 10,000,000:1 or from about 50:1 to 500,000:1. In some embodiments, an amount of about 1 to about 10 ppm, or about 2 ppm to about 5 ppm, of the metathesis catalyst per double bond of the starting composition (i.e., on a mole/mole basis) is used.

In some embodiments, the metathesis reaction is catalyzed by a system containing both a transition and a non-transition metal component. The most active and largest number of catalyst systems are derived from Group VI A transition metals, for example, tungsten and molybdenum.

Multiple, sequential metathesis reaction steps may be employed. For example, the metathesized unsaturated polyol ester product may be made by reacting an unsaturated polyol ester in the presence of a metathesis catalyst to form a first metathesized unsaturated polyol ester product. The first metathesized unsaturated polyol ester product may then be reacted in a self-metathesis reaction to form another metathesized unsaturated polyol ester product. Alternatively, the first metathesized unsaturated polyol ester product may be reacted in a cross-metathesis reaction with a unsaturated polyol ester to form another metathesized unsaturated polyol ester product. Also in the alternative, the transesterified products, the olefins and/or esters may be further metathesized in the presence of a metathesis catalyst. Such multiple and/or sequential metathesis reactions can be performed as many times as needed, and at least one or more times, depending on the processing/compositional requirements as understood by a person skilled in the art. As used herein, a "metathesized unsaturated polyol ester product" may include products that have been once metathesized and/or multiply metathesized. These procedures may be used to form metathesis dimers, metathesis trimers, metathesis tetramers, metathesis pentamers, and higher order metathesis oligomers (e.g., metathesis hexamers, metathesis heptamers, metathesis octamers, metathesis nonamers, metathesis decamers, and higher than metathesis decamers). These procedures can be repeated as many times as desired (for example, from 2 to about 50 times, or from 2 to about 30 times, or from 2 to about 10 times, or from 2 to about 5 times, or from 2 to about 4 times, or 2 or 3 times) to provide the desired metathesis oligomer or polymer which may comprise, for example, from 2 to about 100 bonded groups, or from 2 to about 50, or from 2 to about 30, or from 2 to about 10, or from 2 to about 8, or from 2 to about 6 bonded groups, or from 2 to about 4 bonded groups, or from 2 to about 3 bonded groups. In certain embodiments, it may be desirable to use the metathesized unsaturated polyol ester products produced by cross metathesis of an unsaturated polyol ester, or blend of unsaturated polyol esters, with a C2-C100 olefin, as the reactant in a self-metathesis reaction to produce another metathesized unsaturated polyol ester product. Alternatively, metathesized products produced by cross metathesis of an unsaturated polyol ester, or blend of unsaturated polyol esters, with a C2-C100 olefin can be combined with an unsaturated polyol ester, or blend of unsaturated polyol esters, and further metathesized to produce another metathesized unsaturated polyol ester product.

The metathesis process can be conducted under any conditions adequate to produce the desired metathesis products. For example, stoichiometry, atmosphere, solvent, temperature, and pressure can be selected by one skilled in the art to produce a desired product and to minimize undesirable byproducts. The metathesis process may be conducted under an inert atmosphere. Similarly, if a reagent is supplied as a gas, an inert gaseous diluent can be used. The inert atmosphere or inert gaseous diluent typically is an inert gas, meaning that the gas does not interact with the metathesis catalyst to substantially impede catalysis. For example, particular inert gases are selected from the group consisting of helium, neon, argon, nitrogen, individually or in combinations thereof.

In certain embodiments, the metathesis catalyst is dissolved in a solvent prior to conducting the metathesis reaction. In certain embodiments, the solvent chosen may be selected to be substantially inert with respect to the metathesis catalyst. For example, substantially inert solvents include, without limitation, aromatic hydrocarbons, such as benzene, toluene, xylenes, etc.; halogenated aromatic hydrocarbons, such as chlorobenzene and dichlorobenzene; aliphatic solvents, including pentane, hexane, heptane, cyclohexane, etc.; and chlorinated alkanes, such as dichloromethane, chloroform, dichloroethane, etc. In one particular embodiment, the solvent comprises toluene. The metathesis reaction temperature may be a rate-controlling variable where the temperature is selected to provide a desired product at an acceptable rate. In certain embodiments, the metathesis reaction temperature is greater than about -40° C., greater than about -20° C., greater than about 0° C., or greater than about 10° C. In certain embodiments, the metathesis reaction temperature is less than about 150° C., or less than about 120° C. In one embodiment, the metathesis reaction temperature is between about 10° C. and about 120° C.

The metathesis reaction can be run under any desired pressure. Typically, it will be desirable to maintain a total pressure that is high enough to keep the cross-metathesis reagent in solution. Therefore, as the molecular weight of the cross-metathesis reagent increases, the lower pressure range typically decreases since the boiling point of the cross-metathesis reagent increases. The total pressure may be selected to be greater than about 0.1 atm (10 kPa), in some embodiments greater than about 0.3 atm (30 kPa), or greater than about 1 atm (100 kPa). Typically, the reaction pressure is no more than about 70 atm (7000 kPa), in some embodiments no more than about 30 atm (3000 kPa). A non-limiting exemplary pressure range for the metathesis reaction is from about 1 atm (100 kPa) to about 30 atm (3000 kPa). In certain embodiments it may be desirable to run the metathesis reactions under an atmosphere of reduced pressure. Conditions of reduced pressure or vacuum may be used to remove olefins as they are generated in a metathesis reaction, thereby driving the metathesis equilibrium towards the formation of less volatile products. In the case of a self-metathesis of a natural oil, reduced pressure can be used to remove C₁₂ or lighter olefins including, but not limited to, hexene, nonene, and dodecene, as well as byproducts including, but not limited to cyclohexa-diene and benzene as the metathesis reaction proceeds. The removal of these species can be used as a means to drive the reaction towards the formation of diester groups and cross linked triglycerides.

### Hydrogenation:

In some embodiments, the unsaturated polyol ester is partially hydrogenated before it is subjected to the metathesis reaction. Partial hydrogenation of the unsaturated polyol ester reduces the number of double bonds that are available for in the subsequent metathesis reaction. In some embodiments, the unsaturated polyol ester is metathesized to form a metathesized unsaturated polyol ester, and the metathesized unsaturated polyol ester is then hydrogenated (e.g., partially or fully hydrogenated) to form a hydrogenated metathesized unsaturated polyol ester.

Hydrogenation may be conducted according to any known method for hydrogenating double bond-containing compounds such as vegetable oils. In some embodiments, the unsaturated polyol ester or metathesized unsaturated polyol ester is hydrogenated in the presence of a nickel catalyst that has been chemically reduced with hydrogen to an active state. Commercial examples of supported nickel hydrogenation catalysts include those available under the trade designations "NYSOFACT"^{®}, "NYSOSEL"^{®}, and "NI 5248 D" (from Englehard Corporation, Iselin, N.H.). Additional supported nickel hydrogenation catalysts include those commercially available under the trade designations "PRICAT 9910", "PRICAT 9920", "PRICAT 9908", "PRICAT 9936" (from Johnson Matthey Catalysts, Ward Hill, Mass.).

In some embodiments, the hydrogenation catalyst comprising, for example, nickel, copper, palladium, platinum, molybdenum, iron, ruthenium, osmium, rhodium, or iridium. Combinations of metals may also be used. Useful catalyst may be heterogeneous or homogeneous. In some embodiments, the catalysts are supported nickel or sponge nickel type catalysts.

In some embodiments, the hydrogenation catalyst comprises nickel that has been chemically reduced with hydrogen to an active state (i.e., reduced nickel) provided on a support. In some embodiments, the support comprises porous silica (e.g., kieselguhr, infusorial, diatomaceous, or siliceous earth) or alumina. The catalysts are characterized by a high nickel surface area per gram of nickel.

In some embodiments, the particles of supported nickel catalyst are dispersed in a protective medium comprising hardened triacylglyceride, edible oil, or tallow. In an exemplary embodiment, the supported nickel catalyst is dispersed in the protective medium at a level of about 22 wt. % nickel.

Hydrogenation may be carried out in a batch or in a continuous process and may be partial hydrogenation or complete hydrogenation. In a representative batch process, a vacuum is pulled on the headspace of a stirred reaction vessel and the reaction vessel is charged with the material to be hydrogenated (e.g., RBD soybean oil or metathesized RBD soybean oil). The material is then heated to a desired temperature. Typically, the temperature ranges from about 50 deg. C. to 350 deg. C., for example, about 100 deg. C. to 300 deg. C. or about 150 deg. C. to 250 deg. C. The desired temperature may vary, for example, with hydrogen gas pressure. Typically, a higher gas pressure will require a lower temperature. In a separate container, the hydrogenation catalyst is weighed into a mixing vessel and is slurried in a small amount of the material to be hydrogenated (e.g., RBD soybean oil or metathesized RBD soybean oil). When the material to be hydrogenated reaches the desired temperature, the slurry of hydrogenation catalyst is added to the reaction vessel. Hydrogen gas is then pumped into the reaction vessel to achieve a desired pressure of H2 gas. Typically, the H2 gas pressure ranges from about 15 to 3000 psig, for example, about 15 psig to 90 psig. As the gas pressure increases, more specialized high-pressure processing equipment may be required. Under these conditions the hydrogenation reaction begins and the temperature is allowed to increase to the desired hydrogenation temperature (e.g., about 120 deg. C. to 200 deg. C.) where it is maintained by cooling the reaction mass, for example, with cooling coils. When the desired degree of hydrogenation is reached, the reaction mass is cooled to the desired filtration temperature.

The amount of hydrogenation catalysts is typically selected in view of a number of factors including, for example, the type of hydrogenation catalyst used, the amount of hydrogenation catalyst used, the degree of unsaturation in the material to be hydrogenated, the desired rate of hydrogenation, the desired degree of hydrogenation (e.g., as measure by iodine value (IV)), the purity of the reagent, and the H2 gas pressure. In some embodiments, the hydrogenation catalyst is used in an amount of about 10 wt. % or less, for example, about 5 wt. % or less or about 1 wt. % or less.

After hydrogenation, the hydrogenation catalyst may be removed from the hydrogenated product using known techniques, for example, by filtration. In some embodiments, the hydrogenation catalyst is removed using a plate and frame filter such as those commercially available from Sparkler Filters, Inc., Conroe Tex. In some embodiments, the filtration is performed with the assistance of pressure or a vacuum. In order to improve filtering performance, a filter aid may be used. A filter aid may be added to the metathesized product directly or it may be applied to the filter. Representative examples of filtering aids include diatomaceous earth, silica, alumina, and carbon. Typically, the filtering aid is used in an amount of about 10 wt. % or less, for example, about 5 wt. % or less or about 1 wt. % or less. Other filtering techniques and filtering aids may also be employed to remove the used hydrogenation catalyst. In other embodiments the hydrogenation catalyst is removed using centrifugation followed by decantation of the product.

### Additional optional liquid phase components

Suitable additional liquid phase components for use herein include for example natural oils, synthetic oils, silicone oils, petrolatum, triglycerides, butters or mixtures thereof. As used herein, the term "oil" includes, but is not limited to any non-aqueous substance that is very slightly soluble, preferably practically insoluble in water according to the United States' Pharmacopeia (USP) definition in 31/NF 26 Vol. 2 General Notices, Page Xvii. According to that definition, means that 1000 to 10000 parts of water are needed to dissolve 1 part solute and that more than 10,000 parts of water are needed to dissolve 1 part solute respectively and is liquid at 25°C. Petrolatum may be considered as a lipophilic structurant or a liquid phase due to its complex mixture of component materials. For the purposes of this invention petrolatum is considered as a liquid phase component.

The oil may be selected from natural oil, synthetic oil, silicone oil and mixtures thereof. Non-limiting examples of suitable natural oils include Acetylated Castor Oil, Acetylated Hydrogenated Castor Oil, Actinidia Chinensis (Kiwi), Seed Oil, Adansonia Digitata Oil, Aleurites Moluccana Seed Oil, Anacardium Occidentale (Cashew) Seed Oil, Arachis Hypogaea (Peanut) Oil, Arctium Lappa Seed Oil, Argania Spinosa Kernel Oil, Argemone Mexicana Oil, Avena Sativa (Oat) Kernel Oil, Bertholletia Excelsa Seed Oil, Borago Officinalis Seed Oil, Brassica Campestris (Rapeseed) Seed Oil, Calophyllum Tacamahaca Seed Oil, Camellia Japonica Seed Oil, Camellia Kissi Seed Oil, Camellia Oleifera Seed Oil, Canola Oil, Caprylic/Capric/Lauric Triglyceride, Caprylic/Capric/Linoleic Triglyceride, Caprylic/Capric/My- ristic/Stearic Triglyceride, Caprylic/Capric/Stearic Triglyceride, Caprylic/Capric Triglyceride, Carthamus Tinctorius (Hybrid Safflower) Seed Oil, Carthamus Tinctorius (Safflower) Seed Oil, Carum Carvi (Caraway) Seed Oil, Carya IlIinoensis (Pecan) Seed Oil, Castor Oil Benzoate, Chenopodium Quinoa Seed Oil, Cibotium Barometz Oil, Citrullus Vulgaris (Watermelon) Seed Oil, Cocos Nucifera (Coconut) Oil, Cod Liver Oil, Coffea Arabica (Coffee) Seed Oil, Coix Lacryma-Jobi (Job's Tears) Seed Oil, Corylus Americana (Hazel) Seed Oil, Corylus Avellana (Hazel) Seed Oil, Cucumis Sativus (Cucumber) Oil, Cucurbita Pepo (Pumpkin) Seed Oil, Daucus Carota Sativa (Carrot) Seed Oil, Elaeis Guineensis (Palm) Kernel Oil, Elaeis Guineensis (Palm) Oil, Gossypium (Cotton) Seed Oil, Helianthus Annuus (Hybrid Sunflower) Oil, Helianthus Annuus (Sunflower) Seed Oil, Hippophae Rhamnoides Oil, Human Placental Lipids, Hydrogenated Canola Oil, Hydrogenated Castor Oil, Hydrogenated Castor Oil Laurate, Hydrogenated Castor Oil Triisostearate, Hydrogenated Coconut Oil, Hydrogenated Cottonseed Oil, Hydrogenated C12-18 Triglycerides, Hydrogenated Fish Oil, Hydrogenated Lard, Hydrogenated Menhaden Oil, Hydrogenated Mink Oil, Hydrogenated Olive Oil, Hydrogenated Orange Roughy Oil, Hydrogenated Palm Kernel Oil, Hydrogenated Palm Oil, Hydrogenated Peanut Oil, Hydrogenated Rapeseed Oil, Hydrogenated Shark Liver Oil, Hydrogenated Soybean Oil, Hydrogenated Sunflower Seed Oil, Hydrogenated Tallow, Hydrogenated Vegetable Oil, lsatis Tinctoria Seed Oil, Juglans Regia (Walnut) Seed Oil, Lauric/Palmitic/Oleic Triglyceride, Umnanthes Alba (Meadowfoam) Seed Oil, Unum Usitatissimum (Linseed) Seed Oil, Lupinus Albus Seed Oil, Macadamia Integrifolia Seed Oil, Macadamia Ternifolia Seed Oil, Maleated Soybean Oil, Mangifera Indica (Mango) Seed Oil, Marmot Oil, Melaleuca Alternifolia (Tea Tree) Leaf Oil, Melia Azadirachta Seed Oil, Melissa Officina lis (Balm Mint) Seed Oil, Menhaden Oil, Mink Oil, Moringa pterygosperma Seed Oil, Mortierella Oil, Neatsfoot Oil, Nelumbium Speciosum Flower Oil, Nigella Sativa Seed Oil, Oenothera Biennis (Evening Primrose) Oil, Olea Europaea (Olive) Fruit Oil, Olea Europaea (Olive) Husk Oil, Orange Roughy Oil, Orbignya Cohune Seed Oil, Orbignya Oleifera Seed Oil, Oryza Sativa (Rice) Bran Oil, Oryza Sativa (Rice) Germ Oil, Ostrich Oil, Oxidized Corn Oil, Oxidized Hazel Seed Oil, Papaver Orientale (Poppy) Seed Oil, Passiflora Edulis Seed Oil, Persea Gratissima (Avocado) Oil, Pistacia Vera Seed Oil, Placental Lipids, Prunus Amygdalus Amara (Bitter Almond) Kernel Oil, Prunus Amygdalus Dulcis (Sweet Almond) Oil, Prunus Armeniaca (Apricot) Kernel Oil, Prunus Avium (Sweet Cherry) Seed Oil, Prunus Cerasus (Bitter Cherry) Seed Oil, Prunus Persica (Peach) Kernel Oil, Pyrus Malus (Apple) Oil, Ribes Nigrum (Black Currant) Seed Oil, Ricinus Communis (Castor) Seed Oil, Rosa Canina Fruit Oil, Rosa Moschata Seed Oil, Salmon Oil, Salvia Hispanica Seed Oil, Santalum Album (Sandalwood) Seed Oil, Sesamum Indicum (Sesame) Seed Oil, Shark Liver Oil, Solanum Lycopersicum (Tomato) Seed Oil, Soybean Lipid, Sphingolipids, Taraktogenos Kurzii Seed Oil, Telphairia Pedata Oil, Vegetable Oil, Vitis Vinifera (Grape) Seed Oil, Zea Mays (Corn) Germ Oil, Zea Mays (Corn) Oil, mineral oil and mixtures thereof.

Suitable synthetic oils include hydrocarbons, esters, alkanes, alkenes and mixtures thereof. Non-limiting examples include isopropyl palmitate, isopropyl stearate, isohexadecane, isododecane, polyglyceryl triisostearate and mixtures thereof.

Non-limiting examples of suitable silicone oils include dimethicones (including partial esters of dimethicones and fatty acids derived from natural/synthetic oils), cyclomethicones, phenylated silicones, phenyl trimethicones, trimethyl pentaphenyl trisiloxane, silicone polyether block copolymers and mixtures thereof.

Suitable silicone polyether copolymers may comprise from about 1% to 50%, by weight of polyethylene oxide, from about 20% to about 90% by weight of polypropylene oxide and from about 1% to about 20% by weight of silicone. Preferably the silicone polyether copolymer comprises at least about 40%, more preferably at least about 50%, most preferably at least about 60% by weight of polypropylene oxide. In addition, the silicone polyether copolymer preferably comprises at least about 10%, more preferably from at least about 15%, most preferably from about 15% to 30% by weight of polyethylene oxide. Furthermore, the silicone polyether block copolymer comprises from 1% to 20%, preferably 10% to 20%, more preferably about 15% by weight of silicone.

While silicone polyether block copolymers are known in the art to provide a number of benefits such as foaming, defoaming, wetting, deaeration and lubricity, it has been now been surprisingly found that the selection of silicone block copolymers having from 20% to 90% by weight of polypropylene oxide and from 1% to 50% of polyethylene oxide unexpectedly further provide improved lubrication whilst ensuring the required level of water dispersion and or solubility verses silicone polyether block copolymers having less or no polypropylene oxide and more polyethylene oxide. Moreover, the use of such silicone block copolymers provides improved adhesion to the skin verses alternative materials such as copolymers of polyethylene oxide and polypropylene oxide. Furthermore, the inclusion of 1% to 20% of silicone by weight of the silicone polyether block copolymer surprisingly provides desirable levels of lubrication despite being present at low levels in the polymer.

The copolymers are block copolymers and may preferably have a pendant graft structure. The silicone polyether block copolymer preferably has a ratio of polyethylene oxide units to polypropylene oxide units of from 3.0 to 0.1, preferably from 2.0 to 0.1, more preferably from 0.6 to 0.25. The silicone polyether block copolymer preferably has a ratio of polyethylene oxide units to polypropylene oxide units to silicone units of from 20:65:15.

The silicone polyether copolymer may have a molecular weight of from about 10000 to about 19000, more preferably from about 10000 to 15000. Suitable silicone polyether copolymers are available from Momentive under the Silwets trademark products including L7210, L7602, L7220, L7230, L7500, preferably L7210 and L7602.

Non-limiting examples of commercially available silicone oils include Dow Corning 200 fluid, Dow Corning 244, Dow Corning 245, Dow Corning 344, and Dow Corning 345, (commercially available from Dow Corning Corp.); SF-1204 and SF-1202 Silicone Fluids (commercially available from G.E. Silicones), GE 7207 and 7158 (commercially available from General Electric Co.); and SWS-03314 (commercially available from SWS Silicones Corp.), the Viscasil series (sold by General Electric Company), SF 1075 methyl-phenyl fluid (sold by General Electric Company) and 556 Cosmetic Grade Fluid (sold by Dow Corning Corp.), Silshine 151 (sold by Momentive), PH1555 and PH1560 (sold by Dow Corning) and Silwets such as Silwets 7210, 7230 and 7220 (available from by Momentive).
Suitable triglycerides, may have the following formula: 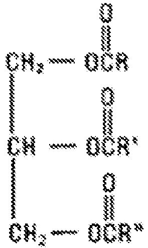 wherein R, R' and R" may be the same as or different from one or both of the others, wherein each of R, R' and R" is a fatty acid and wherein the or each triglyceride is solid at 25°C.

Suitable oils from which triglycerides may be formed from include, but are not limited to, the oils listed herein. Suitable fatty acids for formation of triglycerides include, but are not limited to, Myristoleic acid, Palmitoleic acid, Sapienic acid, Oleic acid, Linoleic acid, α-Linolenic acid ,Arachidonic acid , Eicosapentaenoic acid, Docosahexaenoic acid, Lauric acid (C₁₂), Myristic acid (C₁₄), Palmitic acid (C₁₆), Stearic acid (C₁₈), Arachidic acid (C₂₀) and mixtures thereof.

Specific sources of triglycerides suitable for inclusion herein include Shea Butter, Theobroma Cacao (Cocoa) Seed Butter, Cocoa Butter, Mangifera Indica (Mango) Seed Butter, Kokum Butter and mixtures thereof. Particularly preferred are shea butter, cocoa butter and mixtures thereof.

Preferred liquid phase components may be selected from capric and or caprylic triglycerides, olive oil, shea butter, cocoa butter, petrolatum, isopropyl isostearate, dimethicones, phenylated silicones, silicone polyether block copolymers and mixtures thereof. The silicone polyether block polymers are particularly advantageous as they may facilitate the dispersion of the water soluble polymer in the lipophilic structurant as discussed hereinafter and may also improve lubrication.

### Water soluble polymer

The lubricating member may further comprise from about 1% to about 40% by weight, preferably from about 5% to about 40%, more preferably from about 10% to about 30% and even more preferably from about 20% to about 30% by weight of a water soluble polymer.

Examples of suitable water soluble polymers suitable for use hererin include polyethylene oxide, polyvinyl pyrrolidone, polyacrylamide, polyhydroxymethacrylate, polyvinyl imidazoline, polyethylene glycol, polyvinyl alcohol, polyhydroxyethymethacrylate, quaternary ammonium polymers, guars, celluloses, modified celluloses and mixtures thereof. In some embodiments the water soluble polymers may be selected from polyethylene oxide, polyvinyl pyrrolidone, polyacrylamide, polyhydroxymethacrylate, polyvinyl imidazoline, polyethylene glycol, polyvinyl alcohol, polyhydroxyethymethacrylate, quaternary ammonium polymers and mixtures thereof. In one embodiment, said water soluble polymer is selected from the group consisting of polyethylene oxide, polyethylene glycol, and mixtures thereof

The preferred water soluble polymers are the polyethylene oxides generally known as POLYOX (available from Union Carbide Corporation) or ALKOX^{®} (available from Meisei Chemical Works, Kyoto, Japan). The water soluble polymer, (especially these polyethylene oxides), may have average molecular weights of at least about 20,000, preferably at least about 50,000, more preferably at least about 100,000 or from about 100,000 to about 8 million, preferably about 300,000 to about 5 million, more preferably from about 1 million to about 5 million, even more preferably from about 2 million to about 4 million. One preferred polyethylene oxide comprises a blend of about 40% to 80% of polyethylene oxide having an average molecular weight of about 5 million (e.g. POLYOX COAGULANT) and about 60% to 20% of polyethylene oxide having an average molecular weight of about 300,000 (e.g. POLYOX WSR-N-750). The polyethylene oxide blend may also advantageously contain up to about 10% (for example about 5%) by weight of a low molecular weight (i.e. MW<10,000) polyethylene glycol such as PEG-100.

Suitable water soluble cationic polymers are, for example, cationic cellulose derivatives, for example a quaternized hydroxymethyl cellulose obtainable under the name UCARE POLYMER JR 400^{®} from Dow, hydrophobized quaternized hydroxymethyl cellulose, for example SOFTCAT^{®} SL-5 from Dow, cationic starches, copolymers of diallylammonium salts and acrylamides, quaternized vinylpyrrolidone/vinyl imidazole polymers, for example LUVIQUAT^{®} (BASF), condensation products of polyglycols and amines, quaternized collagen polypeptides, for example lauryldimonium hydroxypropyl hydrolysed collagen (LAMEQUAT^{®}- L/Grünau), quaternised wheat polypeptides, polyethyleneimine, cationic silicone polymers, for example amidomethicones, copolymers of adipic acid and Dimethylaminohydroxypropyldiethy- lenetriamine (CARTARETIN^{®}/Clariant), copolymers of acrylic acid with dimethyldiallylammo- nium chloride (MERQUAT^{®} 550/Chemviron), polyaminopolyamides, as described, for example, in FR-A-2 252840, and the cross-linked water-soluble polymers thereof, cationic chitin derivatives, for example of quaternised chitosan, optionally distributed as microcrystals; condensation products of dihaloalkyls, for example dibromobutane, with bisdialkylamines, for example bisdimethylamino-1,3-propane, cationic guar gum, for example JAGUAR^{®} C-17 from Celanese or N-HANCE^{®} 3196 from Ashland , quaternised ammonium salt polymers, for example MIRAPOL^{®} A-15, MIRAPOL^{®} AD-1, MIRAPOL^{®} AZ-1 from Miranol.

The water soluble polymer is preferably selected so that it is solid at standard ambient temperature and pressure. The water soluble polymer may thus have a melting point of 60°C or greater, preferably 65°C or greater more preferably 70°C or greater. For embodiments comprising more than one water soluble polymer the melting point of each component preferably has a melting point of 60°C or greater, preferably 65°C or greater more preferably 70°C or greater. The melting point is determined according to ASTM D5440-93.

In one embodiment of the invention the melting point of the lipophilic structurant (or mixture, if present) is at least 5°C, preferably at least 10°C less than the melting point of the water soluble polymer, or from 5°C to 45°C less than the melting point of the water soluble polymer.

In another embodiment the water soluble polymer is provided in the form of particles, preferably discrete particles dispersed within the lipophilic structurant. Preferably at least 90%, more preferably at least 95% of said water soluble polymer is in the form of discrete particulates dispersed within said lipophilic structurant. These particulates may have an average particle size of from 50 to 1250 microns, preferably less than 1000 microns. The particles can be readily observed using scanning electron microscopy techniques

While not being bound by theory it is believed that lipophilic structurant having a melting point between about 45°C or greater than 45°C and less than 60°C, enables the water insoluble materials to be melted during the manufacturing process of the lubricating member in a simple hot melt one batch process, but at temperatures which allow the addition of thermally sensitive ingredients such as water soluble polymers without these materials being melted. Moreover it has been surprisingly found that the addition of the water soluble polymer does not require that the polymer is in a liquid form or that it is phase compatible with the lipophilic structurant. In fact is has been surprisingly found that by selecting a water soluble polymer having a melting point above the melting point of the lipophilic structurant, the water soluble material may be added in a solid form, as particulates which are dispersed throughout the lipophilic structurant. It is believed that consequently the water soluble material does not undergo any significant thermal degradation during manufacture thereby increasing its efficacy as a lubricant. Moreover using a particulate dispersion of the water soluble polymer obsoletes the need for high temperature and high shear process step during manufacture in order to incorporate within the lipophilic structurant. The dispersion of the water soluble polymer may be further improved by the incorporation of silicone polyether block polymers as one of the components of the liquid phase.

### Water insoluble polymeric structurant

The lubricating member may comprise less than 5% by weight preferably less than 1% by weight, more preferably is substantially free of a water insoluble polymeric structurant. Whilst not bound by theory the structuring properties of the lubricating member of the present invention are provided by the lipophlic structurant and consequently additional water insoluble polymers are not required. Such water insoluble polymeric structurants include polyethylene (PE), polypropylene, polystyrene (PS), butadiene-styrene copolymer (e.g. medium and high impact polystyrene), polyacetal, acrylonitrile-butadiene-styrene copolymer, ethylene vinyl acetate copolymer, polyurethane, and blends thereof such as polypropylene/polystyrene blend or polystyrene/impact polystyrene blend.

### Optional Ingredients

In some embodiments, the lubricating material may comprise any other ingredients commonly found in commercially available shaving aid members. The lubricating member may therefore contain other conventional shaving aid ingredients, such as low molecular weight water-soluble release enhancing agents such as polyethylene glycol (MW<10,000, e.g., 1-10% by weight PEG-100), water-swellable release enhancing agents such as cross-linked polyacrylics (e.g., 2-7% by weight), colorants, skin feel/care actives, surfactants, soaps (including interrupted soaps), antioxidants, preservatives, emollients, beard softeners, astringents, medicinal agents, plasticizers, additional lubricants, depilatories/keratolytic materials, tackifiers, skin-soothing agents, fragrances, compatibilisers, anti-inflammatory agents, antipruritic/counterirritant mater- ials, dyes, pigments etc. and mixture thereof.

Other optional components may include skin active agents such as, but not limited to oil soluble vitamins, such as vitamin E derivatives, including vitamin E acetate and tocopherol nicotinate; oil-soluble vitamin A derivatives, such as retinyl palmitate; lanolin; ceramides; sterols and sterol esters; salicylic acid; camphor; eucalyptol; essential oils; peppermint oil, Iso E Super [(1-(1,2,3,4,5,6,7,8-octahydro-2,3,8,8-tetramethyl-2-naphthalenyl)ethanone]; and mixtures there- of. Particularly preferred are lanolin, essential oils, peppermint oil, coolants or senates and mixtures thereof. Suitable synthetic coolants include derivatives of or structurally related menthol compounds, i.e., containing the cyclohexane moiety, and derivatized with functional groups including carboxamide, ketal, ester, ether and alcohol. Non-limiting examples include methyl emthylamido oxalate, (under the tradename FRESCOLAT^{®} X-cool available from Symrise), menthyl lactate (such as FRESCOLAT^{®} ML Natural available from Symrise), and Menthyl Pyrrolidone Carboxylate also known as Menthyl PCA (under the tradename QUESTICES^{®} available from Givaudan). Optional components which are liquids are included in determining the total amount of liquid phase present

### Method of manufacture

Another aspect of the invention relates to a method of manufacturing a lubricating member. The method comprises the steps of:
i) Providing a particulate of said water soluble polymer,
ii) Melting said lipophilic structurant, preferably at a temperature of less than about 90°C, more preferably between about 45°C and about 60°C whilst mixing,
iii) Adding said liquid phase and optional ingredients,
iv) Optionally cooling the mixture, preferably to about 60°C or less and then adding said water soluble polymer particles to said melted lipophilic structurant and liquid phase mixture and mixing,
v) Adding other optional ingredients and mixing,
vi) Transferring, for example pouring the resultant mixture into a mould or container,
vii) Optionally cooling to about 25°C.

### Hair Removal Head

According to some embodiments of the invention, the lubricating member finds particular application for hair removal devices. Hair removal devices generally comprise a hair removal head and a handle or grip portion, upon which the hair removal head is mounted. The hair removal device can be manual or power driven and can be used for wet and/or dry application. The hair removal head can include a wide scraping surface such as where the hair removal device is used with a depilatory, or be a razor cartridge or foil where the device is a shaving razor. The hair removal head may be replaceable and/or pivotally connected to a cartridge connecting structure and in turn or independently (e.g. permanently fixed) to a handle. In some embodiments, the cartridge connecting structure includes at least one arm to releasably engage the hair removal head.

The hair removal head typically comprises one or more elongated edges usually positioned between a first and second end, said one or more elongated edges comprising a tip extending towards said first end. Where the hair removal head is a razor cartridge the one or more elongated edges can include blades. For example, U.S. Patent 7,168,173 generally describes a FUSION^{®} razor that is commercially available from The Gillette Company and which includes a razor cartridge with multiple blades. Additionally, the razor cartridge may include a guard as well as a skin engaging member. A variety of razor cartridges can be used in accordance with the present invention. Non limiting examples of suitable razor cartridges, with and without fins, guards, and/or shave aids, include those marketed by The Gillette Company under the FUSION^{®}, VENUS^{®} product lines as well as those disclosed in U.S. Patent Nos. 7,197,825, 6,449,849, 6,442,839, 6,301,785, 6,298,558; 6,161,288, and U.S. Patent Publ. 2008/060201. Those of skill in the art will understand that the lubricating member can be used with any currently marketed system or disposable razor, including those having 2, 3, 4 or 5 blades. In such a case, the hair removal device is a razor, the hair removal head is a razor cartridge and the one or more elongated edges are blades. Another example of a hair removal device is a scraping tool for use with a hair removal composition, i.e. a depilatory.

In some embodiments, said at least one lubricating member is located on the portion of the cartridge that contacts skin during the hair removal process, forward and/or aft of the blades. A feature "forward" of the one or more elongated edges, for example, is positioned so that the surface to be treated with by the hair removal device encounters the feature before it encounters the elongated edges. A feature "aft" of the elongated edge is positioned so that the surface to be treated by the hair removal device encounters the feature after it encounters the elongated edges. Where more than one lubricating member is provided on the hair removal device, they can be the same (identical) or different, in terms of physical shape/structure and/or chemical composition, and one or more of them may comprise the spray coated particulate.

In some particular embodiments, a plurality (e.g. 2, a first and second) of lubricating members may be provided on the hair removal head, with the first skin engaging member comprising the same composition or different. These lubricating members may be placed collectively (for example adjacent to one another) ahead of or behind the elongated edges (e.g. blades on a razor cartridge), including side by side, or separately with one ahead of the elongated edges and the other behind.

The lubricating member may be free standing utilizing a suitable attachment means such as adhesive or may be contained at least partially within a container.

The container typically has a base and at least one side wall extending vertically preferably perpendicular from said base and a skin contacting surface. In a preferred embodiment said container comprises a base and at least 2 side walls, more preferably at least 4 side walls, preferably said walls completely enclosing the base. Typically, each pair of walls are substantially parallel and preferably one pair of walls is substantially parallel to the at least two blades. Alternatively, the base may be enclosed by a one piece single wall. The container may form any shape including substantially rectangular, or oval. The container typically has a front wall adjacent the blades and a rear wall, preferably substantially parallel thereto and furthest from said blades.

The container is preferably further provided with at least one dispensing orifice for dispensing the lubricating member onto the skin during use. In one embodiment the container is provided with a top extending substantially perpendicular from the side wall (s). The container would in such an embodiment typically have a receiving region for receiving the lubricating member. The top may be substantially parallel to the base or it may be provided at an angle such that the distance of the top from the blade plane increases or decreases as the distance of the container from the blades increases. In one embodiment the height of the top of the container increases in distance from the blade plane as the container distance from the blades increases. In an alternative embodiment the height of the top of the container decreases in distance from the blade plane as the container distance from the blade increases.

The orifice may be of any shape and may, for example, have a cross sectional area of from about 0.00324 to about 1.613 cm². Small orifices can also be provided with cross sectional area of from about 0.0324 to about 0.324 cm², or from about 0.0645 to about 0.16135 cm². Larger orifices can have cross sectional areas of from about 0.324 to about 1.613 cm², or from about 0.645 to about 1.29 cm². The container may comprise a single orifice or multiple orifices which may be large and or small. In one embodiment the container comprises at least two orifices. Combinations of small and large orifices can also be provided on the same skin engaging member, or on separate members on the same cartridge, depending on the desired dispense rate and amount of exposure of the lubricating material to water. In one embodiment the top of the container is provide with one preferably two orifices, more preferably two substantially identical orifices adjacent one another.

In some embodiments, at least a portion of said container is not linear for example angled or curvilinear. Curvilinear as defined herein means that at least a portion is curved such that it does not form a straight line. Where at least two containers are provided, they can also be positioned relative to one another such that they do not form a straight line. Alternatively, the curved or angled nature is such that it forms at least a partial ring. A partial ring, as defined herein, means that the structure has at least two curved or angled sections which are concave to form an inner region. The partial ring can also include a curved or angled portion which is positioned convex to said inner region. One or more of said containers may also be positioned relative to one another to form a full ring.

The container can be formed of a variety of materials. The container may, preferably be for example, provided from a non-water soluble material such that it does not degrade or dissolve during normal use. The container typically has sufficient mechanical strength and rigidity to provide adequate mechanical strength to the entire skin engaging member, both as initially produced and after a significant amount of lubricating material has leached out of the container. Alternatively or in addition a further reinforcing member may also be utilized. In some embodiments, the container comprises a base and one or more side walls, forming a receiving region, or channel, onto or into which the lubricating material is placed.

The container may be made of a water-insoluble polymer, particularly a thermoplastic resin. Thermoplastic resins are those materials which can be extruded or molded into a shape and are resilient under normal environmental conditions such as contact with water, even up to normal household hot water temperatures (for example up to 125 °C); normal wear and tear by consumers during use; device assembly and shipping, etc. Thermoplastic resins suitable for use in the carrier include polystyrene, high impact polystyrene (polystyrene-butadiene), polypropylene, filled polypropylene, polyethylene, nylon ethylene vinyl acetate, and blends such as 70% nylon/30% polyethylene oxide, 60% polystyrene/40% polyethylene oxide butadiene styrene copolymer, polyacetal, acrylonitrile-butadiene styrene copolymer, and mixtures thereof. The preferred resins are high impact polystyrene, polystyrene, ethylene vinyl acetate (EVA), and mixtures thereof.

In some embodiments, the cartridge comprises a guard comprising at least one elongated flexible protrusion to engage a user's skin. The at least one flexible protrusion may comprise flexible fins generally parallel to said one or more elongated edges. Said at least one flexible protrusion may additionally or alternatively comprise flexible fins comprising at least one portion which is not generally parallel to said one or more elongated edges. Non-limiting examples of suitable guards include those used in current razor blades and include those disclosed in U.S. Patent Nos. 7,607,230 and 7,024,776; (disclosing elastomeric / flexible fin bars); 2008/0034590 (disclosing curved guard fins); 2009/0049695A1 (disclosing an elastomeric guard having guard forming at least one passage extending between an upper surface and a lower surface). In some embodiments, said lubricating member is positioned on the cartridge aft of the guard and forward of said elongated edge. In another embodiment, the lubricating member is positioned on the cartridge forward of the guard. This embodiment can be particularly useful to deliver the lubricating member prior to contact with the guard.

### TEST METHODS

### Molecular Weight Distribution

The weight average molecular weight (Mw) is measured using gel permeation chromatography (GPC) and multi-angle laser light scattering (MALLS). The GPC/MALLS system used for the analysis is comprised of a Waters Alliance e2695 Separations Module, a Waters 2414 interferometric refractometer, and a Wyatt Heleos II 18 angle laser light scattering detector. The column set used for separation is purchased from TOSOH Biosciences LLC, King of Prussia, PA and included: Guard Column TSKgel G1000Hx-GMHxl-L (Cat # 07113), TSKgel G3000Hxl (Cat # 0016136), TSKgel G2500Hxl (Cat # 0016135), and TSKgel G2000Hxl (Cat # 0016134). Wyatt ASTRA 6 software was used for instrument operation and data analysis. The 90 degree light scattering detection angle is calibrated using filtered, anhydrous toluene. The remaining detection angles are normalized using an isotropic scatterer in THF. To verify instrument performance of the MALLS and RI (refractive index) detectors, a poly(styrene) standard with a known Mw and known dn/dc (in the mobile phase) is run. Acceptable performance of the MALLS and RI detectors gives a calculated Mw within 5% of the reported Mw of the poly(styrene) standard and a mass recovery between 95 and 105%.

To complete the GPC/MALLS analysis, a value of dn/dc is needed. The value of dn/dc is measured as follows. The RI detector is thermostated to 35 degrees Celsius. A series of five concentration standards of the metathesized unsaturated polyol ester in THF is prepared in the range 0.5 mg/ml to 5.5 mg/ml. A THF blank is injected directly into the refractive index detector, followed by each of the metathesized unsaturated polyol ester concentration standards, and ending with another THF blank. The volume of each sample injected is large enough to obtain a flat plateau region of constant differential refractive index versus time; a value of 1.0 ml is typically used. In the ASTRA software, a baseline is constructed from the initial and final THF injections. For each sample, peak limits are defined and the concentrations entered to calculate dn/dc in the ASTRA software. For the metathesized canola oil of Example 2 in THF, a dn/dc value of 0.072 ml/g is obtained.

For the GPC/MALLS analysis of a metathesized unsaturated polyol ester, a total of three samples are evaluated: the metathesized unsaturated polyol ester, a non-metathesized unsaturated polyol ester (glycerol trioleate [122-32-7], Sigma-Aldrich, Milwaukee, WI), and a representative olefin (1-octadecene, [112-88-9], Sigma-Aldrich, Milwaukee, WI). The GPC samples are dissolved in tetrahydrofuran (THF). Concentrations for the metathesized unsaturated polyol ester are approximately 20 mg/ml, and concentrations for the non-metathesized unsaturated polyol ester and olefin are approximately 5 mg/ml. After all the material is dissolved, each solution is filtered by a 0.45 micron nylon filter disk into a GPC autosampler vial for analysis. The GPC column temperature is at room temperature, approximately 25 degrees Celsius. HPLC grade THF is used as the mobile phase and is delivered at a constant flow rate of 1.0 ml/min. The injection volume is 100 microliters and the run time is 40 minutes. Baselines are constructed for all signals. Peak elution limits include metathesized unsaturated polyol ester and non-metathesized unsaturated polyol ester, but exclude later eluting residual olefin. The retention times of the non-metathesized unsaturated polyol ester and olefin were determined from the separate injection runs of both the non-metathesized unsaturated polyol ester and olefin. Baselines and scattering detectors are reviewed.

### Oligomer Index

The oligomer index of the metathesized unsaturated polyol ester is calculated from data that is determined by Supercritical Fluid Chromatography-Fourier Transform Orbital Trapping Mass Spectrometry (SFC-Orbitrap MS). The sample to be analyzed is typically dissolved in methylene chloride or a methylene chloride - hexane mixture at a concentration of 1000 ppm (1 mg/mL). A further 25x-100x dilution is typically made into hexane (for a final concentration of 10-40 ppm). A volume of 2-7.5 µL is typically injected on to a SFC column (for example, a commercially available 3 mm i.d. x 150 mm Ethylpyridine column, 3 µM particle size).

During the chromatography run, the mobile phase is typically programmed from 100% carbon dioxide with a gradient of one percent per minute methanol. The effluent from the column is directed to a mixing tee where an ionization solution is added. The ionization medium is typically 20 mM ammonium formate in methanol at a flow of 0.7 mL/min while the SFC flow is typically 1.6 mL/min into the tee. The effluent from the mixing tee enters the ionization source of the Orbitrap Mass Spectrometer, which is operated in the heated electrospray ionization mode at 320 °C.

In one aspect, a hybrid linear ion trap - Orbitrap mass spectrometer (i.e., the Orbitrap Elite from Thermoelectron Corp.) is calibrated and tuned according to the manufacturer's guidelines. A mass resolution (m/Δm peak width at half height) from 100,000 to 250,000 is typically used. C,H,O compositions of eluting species (typically associated with various cations, e.g., NH₄⁺, H⁺, Na⁺) are obtained by accurate mass measurement (0.1-2 ppm) and are correlated to metathesis products. Also, sub-structures may be probed by linear ion trap "MSⁿ" experiments with subsequent accurate-mass analysis in the Orbitrap, as practiced typically in the art.

The metathesis monomers, dimers, trimers, tetramers, pentamers, and higher order oligomers are fully separated by SFC. The chromatogram based on ion current from the Orbitrap MS may be integrated, as typically practiced in the art, for each of the particular oligomer groups including metathesis monomers, metathesis dimers, metathesis trimers, metathesis pentamers, and each of the higher order oligomers. These raw areas may then be formulated into various relative expressions, based on normalization to 100%. The sum of the areas of metathesis trimers through the highest oligomer detected is divided by the sum of all metathesis species detected (metathesis monomers to the highest oligomer detected). This ratio is called the oligomer index. As used herein, the "oligomer index" is a relative measure of the fraction of the metathesized unsaturated polyol ester which is comprised of trimers, tetramers, pentamers, and higher order oligomers.

### Iodine Value

Another aspect of the invention provides a method to measure the iodine value of the metathesized unsaturated polyol ester. The iodine value is determined using AOCS Official Method Cd 1-25 with the following modifications: carbon tetrachloride solvent is replaced with chloroform (25ml), an accuracy check sample (oleic acid 99%, Sigma-Aldrich; IV = 89.86 ± 2.00 cg/g) is added to the sample set, and the reported IV is corrected for minor contribution from olefins identified when determining the free hydrocarbon content of the metathesized unsaturated polyol ester.

### Free Hydrocarbon Content

Another aspect of this invention provides a method to determine the free hydrocarbon content of the metathesized unsaturated polyol ester. The method combines gas chromatography / mass spectroscopy (GC/MS) to confirm identity of the free hydrocarbon homologs and gas chromatography with flame ionization detection (GC/FID) to quantify the free hydrocarbon present.

Sample Prep: The sample to be analyzed was typically trans-esterified by diluting (e.g. 400:1) in methanolic KOH (e.g. 0.1N) and heating in a closed container until the reaction was complete (i.e. 90°C for 30 min.) then cooled to room temperature. The sample solution could then be treated with 15% boron tri-fluoride in methanol and again heated in a closed vessel until the reaction was complete (i.e. at 60°C for 30 min.) both to acidify (methyl orange - red) and to methylate any free acid present in the sample. After allowing to cool to room temperature, the reaction was quenched by addition of saturated NaCl in water. An organic extraction solvent such as cyclohexane containing a known level internal standard (e.g. 150ppm dimethyl adipate) was then added to the vial and mixed well. After the layers separated, a portion of the organic phase was transferred to a vial suitable for injection to the gas chromatograph. This sample extraction solution was analyzed by GC/MS to confirm identification of peaks matching hydrocarbon retention times by comparing to reference spectra and then by GC/FID to calculate concentration of hydrocarbons by comparison to standard FID response factors.

A hydrocarbon standard of known concentrations, such as 50ppm each, of typically observed hydrocarbon compounds (i.e. 1-dodecene, 1-tridecene, 1-tetradecene, 1-pentadecene, 1-hexadecene, 1-heptadecene, 1-octadecene, dodecane, tridecane, tetradecane, pentadecane, hexadecane, heptadecane and octadecane) was prepared by dilution in the same solvent containing internal standard as was used to extract the sample reaction mixture. This hydrocarbon standard was analyzed by GC/MS to generate retention times and reference spectra and then by GC/FID to generate retention times and response factors.

GC/MS: An Agilent 7890 GC equipped with a split/splitless injection port coupled with a Waters QuattroMicroGC mass spectrometer set up in EI+ ionization mode was used to carry out qualitative identification of peaks observed. A non-polar DB1-HT column (15m x 0.25mm x 0.1um df) was installed with 1.4mL/min helium carrier gas. In separate runs, 1uL of the hydrocarbon standard and sample extract solution were injected to a 300° injection port with a split ratio of 25:1. The oven was held at 40°C for 1 minute then ramped 15C°/minute to a final temperature of 325°C which was held for 10 minutes resulting in a total run time of 30 minutes. The transfer line was kept at 330°C and the temperature of the EI source was 230°C. The ionization energy was set at 70eV and the scan range was 35-550m/z.

GC/FID: An Agilent 7890 GC equipped with a split/splitless injection port and a flame ionization detector was used for quantitative analyses. A non-polar DB1-HT column (5m x 0.25mm x 0.1um df) was installed with 1.4mL/min helium carrier gas. In separate runs, 1uL of the hydrocarbon standard and sample extract solution was injected to a 330° injection port with a split ratio of 100:1. The oven was held at 40°C for 0.5 minutes then ramped at 40C°/minute to a final temperature of 380°C which was held for 3 minutes resulting in a total run time of 12 minutes. The FID was kept at 380°C with 40mL/minute hydrogen gas flow and 450mL/min air flow. Make up gas was helium at 25mL/min. The hydrocarbon standard was used to create a calibration table in the Chemstation Data Analysis software including known concentrations to generate response factors. These response factors were applied to the corresponding peaks in the sample chromatogram to calculate total amount of free hydrocarbon found in each sample.

### EXAMPLES

While particular embodiments of the present invention have been illustrated and described, it would be obvious to those skilled in the art that various other changes and modifications can be made without departing from the spirit and scope of the invention. It is therefore intended to cover in the appended claims all such changes and modifications that are within the scope of this invention.

Non-limiting examples of product formulations disclosed in the present specification are summarized below.

### Example 1: Synthesis of metathesized canola oil

Prior to the metathesis reaction, the RBD (refined, bleached, and deodorized) canola oil is pre-treated by mixing the oil with 2% (by weight) bleaching clay (Filtrol F-160, BASF, Florham Park, NJ) and heating to 120 °C with a nitrogen sweep for 1.5 hours. The oil is cooled to room temperature, filtered through a bed of CELITE@ 545 diatomaceous earth (EMD, Billerica, MA), and stored under inert gas until ready to use.

To a round-bottomed flask, the oil is added and sub-surface sparged with inert gas while mixing and heating to 55 °C. The catalyst is dissolved in 1,2-dichloroethane ([107-06-2], EMD, Billerica, MA) that is stored over 4 Å molecular sieves and sub-surface sparged with inert gas prior to use. After catalyst addition to the reaction flask, a vacuum is applied to remove volatile olefins that are generated. After ~4 hours reaction time, the vacuum is broken and the metathesized unsaturated polyol ester is cooled to room temperature.

The metathesized canola oil is diluted in hexanes ([110-54-3], EMD, Billerica, MA). To the diluted material, 2% bleaching clay (Filtrol F-160, BASF, Florham Park, NJ) is added and mixed for ~6 hours. The oil is filtered through a bed of CELITE^{®} 545 diatomaceous earth. The oil is treated a second time with 2% bleaching clay (Filtrol F-160, BASF, Florham Park, NJ) for ~6 hours. The oil is filtered through a bed of CELITE^{®} 545 diatomaceous earth and then rotary evaporated to concentrate.

The metathesized canola oil is then passed through a wipe film evaporator at 180 °C and <0.5 Torr vacuum to remove olefins up to and including C-18 chain lengths. Representative examples are summarized in the table below.

| **Example** | **Pretreated Canola Oil (g)^{a}** | **Catalyst** | **Catalyst (g)** | **Max Temperature (°C)** | **Max Vacuum (Torr)** |
|---|---|---|---|---|---|
| 1A | 500 | 1^{b} | 0.25 | 61 | 7.9 |
| 1B | 500 | 2^{c} | 0.25 | 62 | 0.6 |

| | | | | | |
|---|---|---|---|---|---|
| ^{a}Canola oil from J. Edwards, Braintree, MA. ^{b}Tricyclohexylphosphine [4,5-dimethyl-1,3-bis(2,4,6-trimethylphenyl)imidazol-2-ylidene] [2-thienylmethylene]ruthenium (II) dichloride [1190427-50-9] available as CatMETium RF-3 from Evonik Corporation, Parsippany, NJ. ^{c}Tricyclohexylphosphine[1,3-bis(2,4,6-trimethylphenyl)imidazol-2-ylidene] [2-thienylmethylene] ruthenium(II) dichloride [1190427-49-6] available as CatMETium RF-2 from Evonik Corporation, Parsippany, NJ. | | | | | |

The samples 1A and 1B are analyzed for weight average molecular weight, iodine value, free hydrocarbon content and oligomer index, using methods described previously, and are found to approximately have the following values:

| **Example** | **Mw (g/mol)** | **Iodine Value (cg/g)** | **Free Hydrocarbon content (wt%)** | **Oligomer Index** |
|---|---|---|---|---|
| 1A | 5,400 | 85 | 0.5 | 0.05 |
| 1B | 3,900 | 85 | 0.5 | 0.04 |

### Example 2: Remetathesis of metathesized unsaturated polyol ester

Metathesized canola oil, sufficiently stripped of residual olefins (176.28 g from Example 1A) is blended with pretreated canola oil (350.96 g, pretreated as described in Example 1) in a round-bottomed flask. The blend is sub-surface sparged with inert gas while mixing and heating to 55 °C. The catalyst is dissolved in 1,2-dichloroethane ([107-06-2], EMD, Billerica, MA) that is stored over 4 Å molecular sieves and sub-surface sparged with inert gas prior to use. After catalyst addition to the reaction flask, a vacuum is applied to remove volatile olefins that are generated. After ~4 hours reaction time, the vacuum is broken and the metathesized unsaturated polyol ester is cooled to room temperature.

The metathesized canola oil is diluted in hexanes ([110-54-3], EMD, Billerica, MA). To the diluted material, 2% bleaching clay (Filtrol F-160, BASF, Florham Park, NJ) is added and mixed for ~6 hours. The oil is filtered through a bed of Celite^{®} 545 diatomaceous earth. The oil is treated a second time with 2% bleaching clay (Filtrol F-160, BASF, Florham Park, NJ) for ~6 hours. The oil is filtered through a bed of CELITE^{®} 545 diatomaceous earth and then rotary evaporated to concentrate.

The remetathesized canola oil is then passed through a wipe film evaporator at 180 °C and <0.5 Torr vacuum to remove olefins up to and including C-18 chain lengths. A representative example is summarized in the table below.

| **Example** | **Oil Blend (g)** | **Catalyst^{a} (g)** | **Max Temperature (°C)** | **Max Vacuum (Torr)** |
|---|---|---|---|---|
| 2 | 500 | 0.27 | 65 | 0.2 |

| | | | | |
|---|---|---|---|---|
| ^{a}Tricyclohexylphosphine [4,5-dimethyl-1,3-bis(2,4,6-trimethylphenyl)imidazol-2-ylidene] [2-thienylmethylene]ruthenium (II) dichloride [1190427-50-9] available as CatMETium RF-3 from Evonik Corporation, Parsippany, NJ. | | | | |

The sample 2 is analyzed for weight average molecular weight, iodine value, free hydrocarbon content and oligomer index, using methods described previously, and is found to approximately have the following values:

| **Example** | **Mw (g/mol)** | **Iodine Value (cg/g)** | **Free Hydrocarbon content (wt%)** | **Oligomer Index** |
|---|---|---|---|---|
| 2 | 13,000 | 80 | 0.5 | 0.07 |

### Example 3: Synthesis of metathesized unsaturated polyol esters

Prior to the metathesis reaction, the RBD (refined, bleached, and deodorized) oil is pre-treated by mixing the oil with 2% (by weight) bleaching clay (Filtrol F-160, BASF, Florham Park, NJ) and heating to 120 °C with a nitrogen sweep for 1.5 hours. The oil is cooled to room temperature, filtered through a bed of CELITE^{®} 545 diatomaceous earth (EMD, Billerica, MA), and stored under inert gas until ready to use.

To a round-bottomed flask, the oil is added and sub-surface sparged with inert gas while mixing and heating to 55 °C. The catalyst is dissolved in 1,2-dichloroethane ([107-06-2], EMD, Billerica, MA) that is stored over 4 Å molecular sieves and sub-surface sparged with inert gas prior to use. After catalyst addition to the reaction flask, a vacuum is applied to remove volatile olefins that are generated. After ~4 hours reaction time, the vacuum is broken and the metathesized unsaturated polyol ester is cooled to room temperature.

The metathesized oil is diluted in hexanes ([110-54-3], EMD, Billerica, MA). To the diluted material, 2% bleaching clay (Filtrol F-160, BASF, Florham Park, NJ) is added and mixed for ~6 hours. The metathesized oil is filtered through a bed of CELITE^{®} 545 diatomaceous earth. The metathesized oil is treated a second time with 2% bleaching clay (Filtrol F-160, BASF, Florham Park, NJ) for ~6 hours. The metathesized oil is filtered through a bed of CELITE^{®} 545 diatomaceous earth and then rotary evaporated to concentrate.

The metathesized unsaturated polyol ester is then passed through a wipe film evaporator at 180 °C and <0.5 Torr vacuum to remove olefins up to and including C-18 chain lengths.
Representative examples are summarized in the table below.

| **Example** | **Starting unsaturated polyol ester** | **Pretreated Oil (g)** | **Catalyst^{a} (g)** | **Max Temperature (°C)** | **Max Vacuum (Torr)** |
|---|---|---|---|---|---|
| 3A | High erucic acid rapeseed oil | 500 | 0.25 | 61 | 7.9 |
| 3B | Blend of High erucic acid rapeseed oil and canola | 500 (250g HEAR oil and 250g canola oil) | 0.25 | 61 | 7.9 |
| | oil, 50/50 by weight | | | | |
| 3C | High oleic soybean oil | 500 | 0.25 | 61 | 7.9 |

| | | | | | |
|---|---|---|---|---|---|
| ^{a}Tricyclohexylphosphine [4,5-dimethyl-1,3-bis(2,4,6-trimethylphenyl)imidazol-2-ylidene] [2-thienylmethylene]ruthenium (II) dichloride [1190427-50-9] available as CatMETium RF-3 from Evonik Corporation, Parsippany, NJ. | | | | | |

### Example 4

Hydrogenations are performed in a T316 stainless steel, 600 ml Parr reactor (Model Number 4563) containing internal cooling coils and a stir shaft with 2 impellers comprised of 4 blades each.

The metathesized unsaturated polyol ester (approximately 200 g) is dissolved in hexanes (120 ml, [110-54-3], EMD, Billerica Ma). To this solution is added a slurry of Nickel on Silica (20 g, [7440-02-0], Catalog #28-1900, Strem Chemicals, Inc., Newburyport, MA). The slurried mixtures is transferred via vacuum to the Parr reactor. The mixture is degassed with several vacuum/nitrogen fill cycles. Then with stirring (800-900 rpm), hydrogen gas (550-650 psig, [1333-74-0], UHP grade, Wright Brothers, Inc., Montgomery, OH) is charged to the reactor. The reaction is heated at 150 °C and hydrogen gas pressure reduction monitored until constant (~12 hours).

The reaction is cooled to 60 °C and drained from the reactor. The reactor is rinsed with methyl tert-butyl ether ([1634-04-4], EMD, Billerica, MA) and combined with the solid hydrogenated metathesized polyol ester. A hot filtration is then performed to remove the catalyst, followed by vacuum to remove all residual solvent. Fully hydrogenated materials are obtained using the method above. Lower hydrogenation levels are obtained by decreasing the reaction temperature to 125 degrees Celsius using 5 grams of catalyst and reducing the reaction time and hydrogen consumed. Iodine Value (IV) is measured, as described elsewhere.

### Example 5

The metathesis monomers, dimers, trimers, tetramers, pentamers, and higher order oligomers from the product in Example 2 are fully separated by SFC using the method described above. The individual SFC fractions are collected and trimers, tetramers, and higher order oligomers are combined. The oligomer index of this sample is about 1.

### Example lubricating member formulations

| **Ingredient** | **Example 1 % w/w** | **Example 2 % w/w** | **Example 3 % w/w** | **Example 4 % w/w** | **Example 5 % w/w** |
|---|---|---|---|---|---|
| Polyox WSR coag | - | 10 | 20 | 10 | - |
| Polyox N60k | 30 | - | - | - | - |
| Silwet L7210 * | 20 | 40 | - | - | 10 |
| Softcat SL5 ** | - | - | 10 | - | - |
| Nhance 3196 *** | - | 10 | - | - | - |
| Petrolatum | - | - | 10 | - | - |
| Methathesized unsaturated polyol ester | 20 | 10- | 30 | 35 | 35 |
| Cetyl alcohol | 30 | - | 25 | 55 | 55 |
| Stearyl alcohol | | 25 | | | |
| Multiwax 180MH # | - | 5 | 5 | - | - |
| Total | 100 | 100 | 100 | | |

| | | | | | |
|---|---|---|---|---|---|
| Suppliers: * Momentive ,** Dow Chemicals, *** Ashland, $ Dow Corning, # Sonnenborn | | | | | |

Formulation Examples 1-5 were prepared as follows:
1. Sanitize all equipment
2. Turn on water bath/ vessel jacket to 85° C
3. Add lipophilic structurants (cetyl alcohol, stearyl alcohol, multiwax 180MH) and stir with overhead stirrer until completely melted
4. Add oil phase ingredients (Silwet, petrolatum, DC200, shea butter, methathesised polyol esters) and mix until fully liquid
5. Reduce heat to 55° C and add powder ingredients (Polyox, Nhance 3196, SoftCat) until fully dispersed.
6. Transfer mixture into a mould
7. Assemble part onto razor cartridge.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. A lubricating member for a hair removal device comprising,
a) a liquid phase, the liquid phase has a melting point of 45°C or less determined according to ASTM-D5440-93, comprising a metathesized unsaturated polyol ester, said metathesized unsaturated polyol ester having one or more of the following properties, measurable in accordance with the description:
(i) a weight average molecular weight of from 5,000 Daltons to 50,000 Daltons measured in accordance with the method as described under *"Molecular Weight Distribution";*
(ii) an oligomer index from greater than 0 to 1 measured in accordance with the method as described under *"Oligomer Index" ;*
(iii) an iodine value of from 30 to 200 measured in accordance with the method as described under "*Iodine Value";*
b) a lipophilic structurant, the lipophilic structurant having a melting point greater than 45°C to less than 60°C determined according to ASTM-D5440-93; and
c) optionally a water soluble polymer, the water soluble polymer having a melting point of 60°C or greater determined according to ASTM-D5440-93.

2. The lubricating member of any preceding claim wherein said metathesized unsaturated polyol ester has a weight average molecular weight of from 5,500 Daltons to 50,000 Daltons.

3. The lubricating member of any preceding claim, wherein said metathesized unsaturated polyol further comprises one or more of the following properties:
(i) a free hydrocarbon content, based on total weight of metathesized unsaturated polyol ester of from 0% to 5% measured in accordance with the method as described under *"Free Hydrocarbon content"*;
(ii) an iodine value of from 30 to 150.

4. The lubricating member according to any preceding claim, wherein said metathesized unsaturated polyol ester has an iodine value of from 10 to 200.

5. The lubricating member according to any preceding claim, wherein said metathesized unsaturated polyol ester has an oligomer index from 0.001 to 1.

6. The lubricating member according to any preceding claim, wherein said composition comprising, based on total composition weight, from 0.1 % to 50% of said metathesized unsaturated polyol ester.

7. The lubricating member according to any preceding claim, wherein the metathesized unsaturated polyol ester is metathesized at least once.

8. The lubricating member according to any preceding claim, wherein said metathesized unsaturated polyol ester is derived from a natural polyol ester and/or a synthetic polyol ester, preferably said natural polyol ester is selected from the group consisting of a vegetable oil, a animal fat, a algae oil and mixtures thereof; and said synthetic polyol ester is derived from a material selected from the group consisting of ethylene glycol, propylene glycol, glycerol, polyglycerol, polyethylene glycol, polypropylene glycol, poly(tetramethylene ether) glycol, pentaerythritol, dipentaerythritol, tripentaerythritol, trimethylolpropane, neopentyl glycol, a sugar, preferably, sucrose, and mixtures thereof.

9. The lubricating member according to any preceding claim, wherein said metathesized unsaturated polyol ester is selected from the group consisting of metathesized Abyssinian oil, metathesized Almond Oil, metathesized Apricot Oil, metathesized Apricot Kernel oil, metathesized Argan oil, metathesized Avocado Oil, metathesized Babassu Oil, metathesized Baobab Oil, metathesized Black Cumin Oil, metathesized Black Currant Oil, metathesized Borage Oil, metathesized Camelina oil, metathesized Carinata oil, metathesized Canola oil, metathesized Castor oil, metathesized Cherry Kernel Oil, metathesized Coconut oil, metathesized Corn oil, metathesized Cottonseed oil, metathesized Echium Oil, metathesized Evening Primrose Oil, metathesized Flax Seed Oil, metathesized Grape Seed Oil, metathesized Grapefruit Seed Oil, metathesized Hazelnut Oil, metathesized Hemp Seed Oil, metathesized Jatropha oil, metathesized Jojoba Oil, metathesized Kukui Nut Oil, metathesized Linseed Oil, metathesized Macadamia Nut Oil, metathesized Meadowfoam Seed Oil, metathesized Moringa Oil, metathesized Neem Oil, metathesized Olive Oil, metathesized Palm Oil, metathesized Palm Kernel Oil, metathesized Peach Kernel Oil, metathesized Peanut Oil, metathesized Pecan Oil, metathesized Pennycress oil, metathesized Perilla Seed Oil, metathesized Pistachio Oil, metathesized Pomegranate Seed Oil, metathesized Pongamia oil, metathesized Pumpkin Seed Oil, metathesized Raspberry Oil, metathesized Red Palm Olein, metathesized Rice Bran Oil, metathesized Rosehip Oil, metathesized Safflower Oil, metathesized Seabuckthorn Fruit Oil, metathesized Sesame Seed Oil, metathesized Shea Olein, metathesized Sunflower Oil, metathesized Soybean Oil, metathesized Tonka Bean Oil, metathesized Tung Oil, metathesized Walnut Oil, metathesized Wheat Germ Oil, metathesized High Oleoyl Soybean Oil, metathesized High Oleoyl Sunflower Oil, metathesized High Oleoyl Safflower Oil, metathesized High Erucic Acid Rapeseed Oil, and mixtures thereof.

10. The lubricating member according to any preceding claim, further comprising:
from 20% to 90%, preferably from 20% to 80% by weight of a lipid phase comprising,
a) from 10% to 70% by weight of the lubricating member of a lipophilic structurant or mixture thereof,
b) from 10% to 70% by weight of the lubricating member of a liquid phase wherein said lubricating member further optionally comprises from 1% to 40% by weight of a water soluble polymer or mixture thereof, preferably wherein said lipophilic structurant has a melting point of from 45°C to 5°C less than the melting point of said water soluble polymer.

11. The lubricating member according to any preceding claim, wherein at least 90% of said water soluble polymer is in the form of discrete particulates dispersed within said lipophilic structurant, or wherein the water soluble polymer comprises a polyethylene oxide polymer.

12. A lubricating member for a razor cartridge according to claim 1, wherein said liquid phase comprises a component selected from natural oil, synthetic oil, natural butters, triglycerides, petrolatum, silicones and mixtures thereof, preferably a material selected from capric and or caprylic triglycerides, olive oil, shea butter, cocoa butter, isopropyl isostearate, petrolatum, dimethicone, phenylated silicones, silicone polyether block polymer and mixtures thereof, preferably a silicone polyether block copolymer or mixtures thereof.

13. A lubricating member for a razor cartridge according to claim 1, wherein said lipophilic structurant is selected from C14-C20 alcohols, microcrystalline wax, stearylox-ytrimethylsilane and mixtures thereof, and is preferably selected from cetyl alcohol, stearyl alcohol or mixtures thereof.

14. A lubricating member for a razor cartridge according to claim 1, comprising from 25% to 35% of said lipophilic structurant, from 10% to 40% of said liquid phase and from 20% to 30% of said water soluble polymer and wherein said member comprises less than 5% by weight, preferably less than 1% by weight, more preferably is substantially free of a water insoluble polymeric structurant.

15. A hair removal cartridge comprising a lubricating member according to any one of the preceding claims.

## Patentansprüche

1. Gleitmittelelement für eine Haarentfernungsvorrichtung, umfassend,
a) eine Flüssigphase, wobei die Flüssigphase einen Schmelzpunkt von 45 °C oder weniger aufweist, bestimmt gemäß ASTM-D5440-93, umfassend einen metathesierten ungesättigten Polyolester, wobei der metathesierte ungesättigte Polyolester eine oder mehrere der folgenden Eigenschaften aufweist, die gemäß der Beschreibung messbar sind:
(i) ein durchschnittliches Molekulargewicht von 5.000 Dalton bis 50.000 Dalton, gemessen gemäß dem Verfahren, wie unter *"Molekulargewichtsverteilung"* beschrieben;
(ii) einen Oligomerindex von größer als 0 bis 1, gemessen gemäß dem Verfahren, wie unter "*Oligomerindex"* beschrieben;
(iii) einen lodwert von 30 bis 200, gemessen gemäß dem Verfahren, wie unter *"lodwert"* beschrieben;
b) ein lipophiles Strukturmittel, wobei das lipophile Strukturmittel einen Schmelzpunkt von größer als 45 °C bis weniger als 60 °C aufweist, bestimmt gemäß ASTM-D5440-93; und
c) wahlweise ein wasserlösliches Polymer, wobei das wasserlösliche Polymer einen Schmelzpunkt von 60 °C oder größer aufweist, bestimmt gemäß ASTM-D5440-93.

2. Gleitmittelelement nach einem der vorstehenden Ansprüche, wobei der metathesierte ungesättigte Polyolester ein durchschnittliches Molekulargewicht von 5.500 Dalton bis 50.000 Dalton aufweist.

3. Gleitmittelelement nach einem der vorstehenden Ansprüche, wobei der metathesierte ungesättigte Polyolester eine oder mehrere der folgenden Eigenschaften umfasst:
(i) einen freien Kohlenwasserstoffgehalt, basierend auf dem Gesamtgewicht von metathesiertem ungesättigtem Polyolester von 0 % bis 5 %, gemessen gemäß dem Verfahren, wie unter *"Freier Kohlenwasserstoffgehalt"* beschrieben;
(ii) einen lodwert von 30 bis 150.

4. Gleitmittelelement nach einem der vorstehenden Ansprüche, wobei der metasthesierte ungesättigte Polyolester einen lodwert von 10 bis 200 aufweist.

5. Gleitmittelelement nach einem der vorstehenden Ansprüche, wobei der metasthesierte ungesättigte Polyolester einen Oligomerindex von 0,001 bis 1 aufweist.

6. Gleitmittelelement nach einem der vorstehenden Ansprüche, wobei die Zusammensetzung, basierend auf dem Gesamtzusammensetzungsgewicht, zu 0,1 % bis 50 % den metathesierten ungesättigten Polyolester umfasst.

7. Gleitmittelelement nach einem der vorstehenden Ansprüche, wobei der metathesierte ungesättigte Polyolester mindestens einmal metathesiert ist.

8. Zusammensetzung nach einem der vorstehenden Ansprüche, wobei der metathesierte ungesättigte Polyolester von einem natürlichen Polyolester und/oder einem synthetischen Polyolester abgeleitet ist, wobei vorzugsweise der natürliche Polyolester ausgewählt ist aus der Gruppe bestehend aus einem Pflanzenöl, einem tierischen Fett, einem Algenöl und Mischungen davon; und wobei der synthetische Polyolester von einem Material abgeleitet ist, das ausgewählt ist aus der Gruppe bestehend aus Ethylenglykol, Propylenglykol, Glycerin, Polyglycerin, Polyethylenglykol, Polypropylenglykol, Poly(tetramethylenether)glykol, Pentaerythrit, Dipentaerythrit, Tripentaerythrit, Trimethylolpropan, Neopentylglykol, einem Zucker, vorzugsweise Saccharose, und Mischungen davon.

9. Zusammensetzung nach einem der vorstehenden Ansprüche, wobei der metathesierte ungesättigte Polyolester ausgewählt ist aus der Gruppe bestehend aus metathesiertem Krambenöl, metathesiertem Mandelöl, metathesiertem Aprikosenöl, metathesiertem Aprikosenkernöl, metathesiertem Arganöl, metathesiertem Avocadoöl, metathesiertem Babassuöl, metathesiertem Baobaböl, metathesiertem Schwarzkümmelöl, metathesiertem schwarzem Johannisbeeröl, metathesiertem Borretschöl, metathesiertem Leindotteröl, metathesiertem äthiopischem Senföl, metathesiertem Canolaöl, metathesiertem Rizinusöl, metathesiertem Kirschkernöl, metathesiertem Kokosnussöl, metathesiertem Maisöl, metathesiertem Baumwollsamenöl, metathesiertem Echiumöl, metathesiertem Nachtkerzenöl, metathesiertem Flachsöl, metathesiertem Traubenkernöl, metathesiertem Pampelmusenkernöl, metathesiertem Haselnussöl, metathesiertem Hanfsamenöl, metathesiertem Jatrophaöl, metathesiertem Jojobaöl, metathesiertem Kukuinussöl, metathesiertem Leinsamenöl, metathesiertem Macadamianussöl, metathesiertem Wiesenschaumkrautöl, metathesiertem Moringaöl, metathesiertem Neemöl, metathesiertem Olivenöl, metathesiertem Palmöl, metathesiertem Palmkernöl, metathesiertem Pfirsichkernöl, metathesiertem Erdnussöl, metathesiertem Pecannussöl, metathesiertem Hellerkrautöl, metathesiertem Perillaöl, metathesiertem Pistazienöl, metathesiertem Granatapfelkernöl, metathesiertem Pongamiaöl, metathesiertem Kürbiskernöl, metathesiertem Himbeeröl, metathesiertem rotem Palmolein, metathesiertem Reiskleieöl, metathesiertem Hagebuttenöl, metathesiertem Safloröl, metathesiertem Sanddornfruchtöl, metathesiertem Sesamöl, metathesiertem Sheaolein, metathesiertem Sonnenblumenöl, metathesiertem Sojabohnenöl, metathesiertem Tonkabohnenöl, metathesiertem Tungöl, metathesiertem Walnussöl, metathesiertem Weizenkeimöl, metathesiertem Sojabohnenöl mit hohem Ölsäuregehalt, metathesiertem Sonnenblumenöl mit hohem Ölsäuregehalt, metathesiertem Safloröl mit hohem Ölsäuregehalt, metathesiertem Rapsöl mit hohem Erucasäuregehalt und Mischungen davon.

10. Gleitmittelelement nach einem der vorstehenden Ansprüche, ferner umfassend:
zu 20 Gew.-% bis 90 Gew.-%, vorzugsweise zu 20 Gew.-% bis 80 Gew.-% eine Lipidphase, umfassend,
a) zu 10 Gew.-% bis 70 Gew.-% des Gleitmittelelements ein lipophiles Strukturmittel oder eine Mischung davon,
b) zu 10 Gew.-% bis 70 Gew.-% des Gleitmittelelements eine Flüssigphase, wobei das Gleitmittel ferner wahlweise zu 1 Gew.-% bis 40 Gew.-% ein wasserlösliches Polymer oder eine Mischung davon umfasst, vorzugsweise wobei das lipophile Strukturmittel einen Schmelzpunkt aufweist, der 45 °C bis 5 °C weniger als der Schmelzpunkt des wasserlöslichen Polymers ist.

11. Gleitmittelelement nach einem der vorstehenden Ansprüche, wobei mindestens 90 % des wasserlöslichen Polymers in der Form von diskreten Partikeln vorliegen, die innerhalb des lipophilen Strukturmittels dispergiert sind, oder wobei das wasserlösliche Polymer ein Polyethylenoxidpolymer umfasst.

12. Gleitmittelelement für eine Rasierklingeneinheit nach Anspruch 1, wobei die Flüssigphase eine Komponente ausgewählt aus natürlichem Öl, synthetischem Öl, natürlicher Butter, Triglyceriden, Petrolatum, Silikonen und Mischungen davon umfasst, vorzugsweise ein Material ausgewählt aus Caprin- und/oder Capryltriglyceriden, Olivenöl, Sheabutter, Kakaobutter, Isopropylisostearat, Petrolatum, Dimethicon, phenylierten Silikonen, Silikonpolyetherblockpolymer und Mischungen davon, vorzugsweise ein Silikonpolyetherblockcopolymer oder Mischungen davon.

13. Gleitmittelelement für eine Rasierklingeneinheit nach Anspruch 1, wobei das lipophile Strukturmittel ausgewählt ist aus C14-C20-Alkoholen, mikrokristallinem Wachs, Stearyloxytrimethylsilan und Mischungen davon und vorzugsweise ausgewählt ist aus Cetylalkohol, Stearylalkohol oder Mischungen davon.

14. Gleitmittelelement für eine Rasiererklingeneinheit nach Anspruch 1, umfassend zu 25 % bis 35 % das lipophile Strukturmittel, zu 10 % bis 40 % die Flüssigphase und zu 20 % bis 30 % das wasserlösliche Polymer, und wobei das Element weniger als 5 Gew.-%, vorzugsweise weniger als 1 Gew.-% umfasst, und stärker bevorzugt im Wesentlichen frei von einem wasserunlöslichen polymeren Strukturmittel ist.

15. Haarentfernungseinheit, umfassend ein Gleitmittelelement nach einem der vorstehenden Ansprüche.

## Revendications

1. Élément lubrifiant pour un dispositif d'épilation comprenant,
a) une phase liquide, la phase liquide a un point de fusion de 45 °C ou moins déterminé selon la norme ASTM-D5440-93, comprenant un ester de polyol insaturé métathétisé, ledit ester de polyol insaturé métathétisé ayant une ou plusieurs des propriétés suivantes, mesurables conformément à la description :
(i) une masse moléculaire moyenne en poids allant de 5 000 Daltons à 50 000 Daltons, mesurée conformément au procédé tel que décrit dans « Distribution de poids moléculaire » ;
(ii) un indice d'oligomère allant de plus de 0 à 1 mesuré conformément au procédé tel que décrit dans « Indice des oligomères » ;
(iii) une valeur d'iode allant de 30 à 200 mesurée conformément au procédé tel que décrit dans « Valeur d'iode » ;
b) un structurant lipophile, le structurant lipophile ayant un point de fusion supérieur à 45 °C et inférieur à 60 °C déterminé selon la norme ASTM-D5440-93 ; et
c) éventuellement un polymère soluble dans l'eau, le polymère soluble dans l'eau ayant un point de fusion de 60 °C ou plus, déterminé selon la norme ASTM-D5440-93.

2. Élément lubrifiant selon l'une quelconque revendication précédente, dans lequel ledit ester de polyol insaturé ayant subi une métathèse a un poids moléculaire moyen en poids de 5 500 Daltons à 50 000 Daltons.

3. Élément lubrifiant selon l'une quelconque revendication précédente, dans lequel ledit ester de polyol insaturé ayant subi une métathèse comprend une ou plusieurs des propriétés suivantes :
(i) une teneur en hydrocarbure libre, sur la base du poids total d'ester de polyol insaturé ayant subi une métathèse allant de 0 % à 5 %, mesuré conformément au procédé tel que décrit dans « Teneur en hydrocarbures libres » ;
(iii) un indice d'iode allant de 30 à 150.

4. Élément lubrifiant selon l'une quelconque revendication précédente, dans lequel ledit ester de polyol insaturé ayant subi une métathèse a une valeur d'iodine allant d'environ 10 à 200.

5. Élément lubrifiant selon l'une quelconque revendication précédente, dans lequel ledit ester de polyol insaturé ayant subi une métathèse a un indice d'oligomère allant d'environ 0,001 à 1.

6. Élément lubrifiant selon l'une quelconque revendication précédente, dans lequel ladite composition comprenant, sur la base du poids total de la composition, de 0,1 % à 50 % dudit ester de polyol insaturé ayant subi une métathèse.

7. Élément lubrifiant selon l'une quelconque revendication précédente, dans lequel l'ester de polyol insaturé ayant subi une métathèse subit une métathèse au moins une fois.

8. Élément lubrifiant selon l'une quelconque revendication précédente, dans lequel ledit ester de polyol insaturé ayant subi une métathèse est issu d'un ester de polyol naturel et/ou d'un ester de polyol synthétique, de préférence ledit ester de polyol naturel est choisi dans le groupe constitué d'une huile végétale, d'une graisse animale, d'une huile d'algues et de leurs mélanges ; et ledit ester de polyol synthétique est issu d'un matériau choisi dans le groupe constitué d'éthylène glycol, propylène glycol, glycérol, polyglycérol, polyéthylène glycol, polypropylène glycol, poly(tétraméthylène éther) glycol, pentaérythritol, dipentaérythritol, tripentaérythritol, triméthylolpropane, néopentylglycol, un sucre, de préférence, du saccharose, et leurs mélanges.

9. Élément lubrifiant selon l'une quelconque revendication précédente, dans lequel ledit ester de polyol insaturé obtenu par métathèse est choisi dans le groupe constitué d'huile d'abyssinie obtenue par métathèse, huile d'amande obtenue par métathèse, huile d'abricot obtenue par métathèse, huile de noyau d'abricot obtenue par métathèse, huile d'argan obtenue par métathèse, huile d'avocat obtenue par métathèse, huile de babassu obtenue par métathèse, huile de baobab obtenue par métathèse, huile de cumin noir obtenue par métathèse, huile de cassis obtenue par métathèse, huile de bourrache obtenue par métathèse, huile de cameline obtenue par métathèse, huile de carinata obtenue par métathèse, huile de colza obtenue par métathèse, huile de ricin obtenue par métathèse, huile de noyaux de cerise obtenue par métathèse, huile de coco obtenue par métathèse, huile de maïs obtenue par métathèse, huile de graines de coton obtenue par métathèse, huile d'échium obtenue par métathèse, huile d'onagre obtenue par métathèse, huile de graines de lin obtenue par métathèse, huile de pépins de raisin obtenue par métathèse, huile de pépins de pamplemousse obtenue par métathèse, huile de noisette obtenue par métathèse, huile de graines de chanvre obtenue par métathèse, huile de jatropha obtenue par métathèse, huile de jojoba obtenue par métathèse, huile de noix de Kukui obtenue par métathèse, huile de lin obtenue par métathèse, huile de noix de macadamia obtenue par métathèse, huile de graines d'écume des prés obtenue par métathèse, huile de moringa obtenue par métathèse, huile de margousier obtenue par métathèse, huile d'olive obtenue par métathèse, huile de palme obtenue par métathèse, huile de palmiste obtenue par métathèse, huile de noyaux de pêche obtenue par métathèse, huile d'arachide obtenue par métathèse, huile de noix de pécan obtenue par métathèse, huile de tabouret des champs obtenue par métathèse, huile de graines de périlla obtenue par métathèse, huile de pistache obtenue par métathèse, huile de pépins de grenade obtenue par métathèse, huile de pongamia obtenue par métathèse, huile de pépins de courge obtenue par métathèse, huile de framboise obtenue par métathèse, oléine de palme rouge obtenue par métathèse, huile de son de riz obtenue par métathèse, huile de rose musquée obtenue par métathèse, huile de carthame obtenue par métathèse, huile de fruit d'argousier obtenue par métathèse, huile de graines de sésame obtenue par métathèse, oléine de karité obtenue par métathèse, huile de tournesol obtenue par métathèse, huile de soja obtenue par métathèse, huile de fève tonka obtenue par métathèse, huile de tung obtenue par métathèse, huile de noix obtenue par métathèse, huile de germe de blé obtenue par métathèse, huile de soja à haute teneur en oléoyle obtenue par métathèse, huile de tournesol à haute teneur en oléoyle obtenue par métathèse, huile de carthame à haute teneur en oléoyle obtenue par métathèse, huile de colza à haute teneur en acide érucique obtenue par métathèse, et leurs mélanges.

10. Élément lubrifiant selon l'une quelconque revendication précédente, comprenant en outre :
de 20 % à 90 %, de préférence de 20 % à 80 % en poids d'une phase lipide, comprenant :
de 10 % à 70 %, en poids de l'élément de lubrification, d'un structurant lipophile ou d'un mélange de celui-ci,
b) de 10 % à 70 % en poids de l'élément lubrifiant d'une phase liquide, dans lequel ledit élément lubrifiant comprend en outre éventuellement de 1 % à 40 % en poids d'un polymère soluble dans l'eau ou d'un mélange de ceux-ci, de préférence dans lequel ledit structurant lipophile a un point de fusion de 45 °C à 5 °C inférieur au point de fusion dudit polymère soluble dans l'eau.

11. Élément lubrifiant selon l'une quelconque revendication précédente, dans lequel au moins 90 % dudit polymère hydrosoluble se présente sous la forme de particules discrètes dispersées dans ledit structurant lipophile, ou dans lequel le polymère hydrosoluble comprend un polymère d'oxyde de polyéthylène.

12. Élément lubrifiant pour une cartouche de rasoir selon la revendication 1, dans lequel ladite phase liquide comprend un composant choisi parmi les huiles naturelles, les huiles synthétiques, les beurres naturels, les triglycérides, le pétrolatum, les silicones et leurs mélanges, de préférence un matériau choisi parmi les triglycérides capriques et ou capryliques, huile d'olive, beurre de karité, beurre de cacao, isostéarate d'isopropyle, pétrolatum, diméthicone, silicones phénylées, polymère à blocs de polyéther de silicone et leurs mélanges, de préférence un copolymère à blocs de polyéther de silicone ou leurs mélanges.

13. Élément de lubrification pour une cartouche de rasoir selon la revendication 1, dans lequel ledit structurant lipophile est choisi parmi des alcools en C14 à C20, de la cire microcristalline, du stéaryloxytriméthylsilane et des mélanges de ceux-ci, et est de préférence choisi parmi l'alcool cétylique, l'alcool stéarylique et des mélanges de ceux-ci.

14. Élément de lubrification pour une cartouche de rasoir selon la revendication 1, comprenant de 25 % à 35 % dudit structurant lipophile, de 10 % à 40 % de ladite phase liquide et de 20 % à 30 % dudit polymère soluble dans l'eau et dans lequel ledit élément comprend moins de 5 % en poids, de préférence moins de 1 % en poids, plus préférablement est substantiellement exempt d'un structurant polymérique insoluble dans l'eau.

15. Cartouche d'épilation comprenant un élément de lubrification selon l'une quelconque des revendications précédentes.
